# EUROPEAN PATENT SPECIFICATION

(11) **EP 2 140 027 B1**
(45) Date of publication and mention of the grant of the patent: **18.07.2012**
(21) Application number: 07869055.9
(22) Date of filing: 07.12.2007
(51) Int. Cl.: C12Q 1/68

(54) **NON-INVASIVE PRENATAL GENETIC SCREEN**
NICHTINVASIVER PRÄNATALER GENETISCHER SCREEN-TEST
DEPISTAGE GENETIQUE PRENATAL NON INVASIF

(30) Priority: 07.12.2006 US 869090 P
(43) Date of publication of application: 06.01.2010
(73) Proprietor: Novartis AG, 4056 Basel (CH)
(72) Inventor: BHATT, Ram, San Diego, CA 92130 (US); FAN, Wen-Hua, San Diego, CA 92126 (US); TIM, Roger, La Jolla, CA 92037 (US); BISCHOFF, Farideh, Sugar Land, TX 77479 (US)
(74) Representative: Hackney, Nigel John
(86) International application number: PCT/US2007/086862
(87) International publication number: WO 2008/070862

(56) References cited:
- WO-A1-98/54364
- WO-A2-99/14375
- US-A1- 2005 123 914
- LI YING ET AL: "Size separation of circulatory DNA in maternal plasma permits ready detection of fetal DNA polymorphisms" CLINICAL CHEMISTRY, vol. 50, no. 6, 2004, pages 1002-1011, XP002510472 ISSN: 0009-9147
- ADINOLFI M ET AL: "First trimester prenatal diagnosis using transcervical cells: an evaluation" HUMAN REPRODUCTION UPDATE, OXFORD UNIVERSITY PRESS, OXFORD, GB LNKD- DOI:10.1093/HUMUPD/3.4.383, vol. 3, no. 4, 1 January 1997 (1997-01-01) , pages 383-392, XP002986611 ISSN: 1355-4786

## Description

### FIELD OF THE INVENTION

This invention relates generally to the isolation of fetal nucleic acid and prenatal screening or testing of genetic and chromosomal abnormalities.

### BACKGROUND OF THE INVENTION

Prenatal testing or screening is usually performed to determine the gender of the fetus or to detect genetic disorders and/or chromosomal abnormalities in the fetus during pregnancy. As of today, over 4000 genetic disorders, caused by one or more faulty genes, have been recognized. Some examples include Cystic Fibrosis, Huntington's Disease, Beta Thalassaemia, Myotonic Dystrophy, Sickle Cell Anemia, Porphyria, and Fragile-X-Syndrome. Chromosomal abnormality is caused by aberrations in chromosome numbers, duplication or absence of chromosomal material, and by defects in chromosome structure. Some examples of chromosomal abnormalities are trisomies, namely trisomy 16, a major cause of miscarriage in the first trimester, trisomy 21 (Down syndrome), trisomy 13 (Patau syndrome), trisomy 18 (Edwards syndrome), Klinefelter's syndrome (47, XXY), (47, XYY), and (47, XXX); the absence of chromosomes (monosomy), *e.g*., Turner syndrome (45, X0); chromosomal translocations, deletions and/or microdeletions, *e.g*., Robertsonian translocation, Angelman syndrome, DiGeorge syndrome and Wolf-Hirschhorn Syndrome.

Currently available prenatal genetic tests usually involve invasive procedures. For example, chorionic villous sampling (CVS) performed on a pregnant woman around 10-12 weeks into the pregnancy and amniocentesis performed at around 14-16 weeks all contain invasive procedures to obtain the sample for testing chromosomal abnormalities in a fetus. Fetal cells obtained via these sampling procedures are usually tested for chromosomal abnormalities using cytogenetic or fluorescent in situ hybridization (FISH) analyses.

While these procedures can be useful for detecting chromosomal aberrations, they have been shown to be associated with the risk of miscarriage. Therefore amniocentesis or CVS is only offered to women perceived to be at increased risk, including those of advanced maternal age (>35 years), those with abnormal maternal serum screening or those who have had a previous fetal chromosomal abnormality. As a result of these tests the percentage of women over the age of 35 who give birth to babies with chromosomal aberrations such as Down syndrome has drastically reduced. However, lack of appropriate or relatively safe prenatal testing or screening for the majority of pregnant women has resulted in about 80% of Down syndrome babies born to women under 35 years of age.

Thus there is a need for diagnostic screening tests for the general population of pregnant women, especially tests directed to identifying fetal chromosomal aberrations as well as other genetic variations or defects.
Li et al (Clinical Chemistry (2004) 50(6):1002-1011) describe analysis of fetal DNA in maternal plasma.
Adinolfi and Sherlock (Human Reproduction Update (1997) 3(4):383-392) disclose that human trophoblastic cells can be retrieved by minimally invasive procedures from the endocervical canal between 6 and 15 weeks gestation, and that the incidence with which fetal cells can be detected in transcervical cell (TCC) samples varies according to the method of collection and the molecular techniques employed for their identification.
WO98/54364 describes non-invasive methods of detecting the presence of specific nucleic acid sequences by analyzing urine samples for the presence of nucleic acids that have crossed the kidney barrier, including methods of detecting specific fetal nucleic acid sequences by analyzing maternal urine for the presence of fetal nucleic acids.

### SUMMARY OF THE INVENTION

The present invention is based, in part, on the discovery that cervical mucous is a good natural reservoir for migrated placental cells, *e.g*., fetal cells as well as for isolating fetal nucleic acids. Accordingly the present disclosure provides methods and kits useful for testing or screening for genetic abnormalities in fetuses using fetal nucleic acids isolated from cervical mucus samples. In addition, the present disclosure provides primers and probes useful for nucleic acid amplification of, *e.g*., genetic markers, especially using relatively small size amplicons in fetal genetic screening.

In one embodiment of the invention, it provides a method for conducting a genetic test of a fetus. The method comprises isolating, by size fractionation, a nucleic acid sample from a cervical mucus sample obtained from a female subject containing the fetus, wherein the nucleic acid sample consists essentially of polynucleotides in a size ranging from about 50 base pairs to about 300 base pairs and wherein the result of a genetic test on the nucleic acid sample is indicative of a genetic composition of the fetus.

In another embodiment of the invention, it provides a method of isolating a fetal nucleic acid sample. The method comprises isolating, by size fractionation, a nucleic acid sample consisting essentially of polynucleotides of about 50 base pairs to about 300 base pairs in length from a cervical mucus sample obtained from a female subject containing the fetus.

Also discloses is a genetic testing kit suitable for testing the genetic composition of a fetus. The kit comprises a pair of primers suitable for amplifying a desired allele or genetic marker, wherein the amplified nucleotide fragment is less than about 200 base pairs and wherein the desired allele is not uniquely associated with the Y chromosome. In other embodiments, the kit comprises an isolated DNA sample from a cervical mucus sample obtained from a female subject containing the fetus. The DNA sample consists essentially of polynucleotides in a size ranging from about 50 base pairs to about 200 base pairs.

Also disclosed is an isolated DNA sample useful for genetic testing of a fetus. The DNA sample can be obtained by isolating DNA fragments in a size ranging from about 50 base pairs to about 200 base pairs from a cervical mucus sample obtained from a female subject containing the fetus.

### BRIEF DESCRIPTION OF THE FIGURES

FIG. 1 shows the size fractionation of total DNA obtained from cervical mucus on a 10% polyacrylamide gel. "Band A," corresponding to a polynucleotide length of around 50-200 base pairs contains fetal DNA.

FIG. 2 shows an example of PCR electropherogram demonstrating that in one experiment the fetal signals match between fetal tissue DNA and the 50-200 bp fragment of DNA isolated from a cervical mucus sample.

FIG. 3 shows another example of PCR electropherogram demonstrating that in another experiment the fetal signals match between fetal tissue DNA and the 50-200 bp fragment of DNA isolated from a cervical mucus sample.

### DETAILED DESCRIPTION OF THE INVENTION

It is the discovery of the present invention that cervical mucus samples can be a great source for fetal cells as well as fetal nucleic acids. Accordingly, the present disclosure provides methods, reagents and kits useful for testing or screening fetus for genetic abnormalities using nucleic acids isolated from cervical mucus samples.

In addition, the present disclosure provides primers and probes useful for nucleic acid amplification, *e.g*., of genetic markers, especially using relatively small size amplicons in fetal genetic screening.

According to one aspect of the present invention, it provides methods for conducting genetic tests of a fetus by isolating one or more nucleic acid samples from one or more cervical mucus samples obtained from a female subject containing the fetus. In general, the nucleic acid sample useful for the methods of the present invention can be a DNA sample, RNA sample, or a combination thereof including any DNA, cDNA, or RNA derived from one or more nucleic acid samples isolated from one or more cervical mucus samples.

In one embodiment, the nucleic acid sample useful for the methods of the present invention is a DNA sample. In another embodiment, the nucleic acid sample useful for the methods of the present invention is substantially free of proteins or polypeptides. The nucleic acid sample useful for the methods of the present invention is isolated by any known or later discovered size fractionation method including, but not limited to, gel electrophoresis, capillary electrophoresis, size exclusion matrixes, and size fractionation columns.

The nucleic acid sample useful for the methods of the present invention is in a size range representative of, or substantially associated, with fetal nucleic acid. In another embodiment, the nucleic acid sample useful for the methods of the present invention is in a size range substantially free of nucleic acid from the host of the fetus. For example, the nucleic acid sample useful for the methods of the present invention can be in a size range from about 50 to about 300 base pairs, from about 50 to about 250 base pairs, from about 50 to about 200 base pairs, from about 50 to about 150 base pairs, or from about 50 to about 100 base pairs or a combination thereof, and optionally, does not contain a substantial amount, *e.g*., more than 0.5%, 1%, 2%, 3%, 4%, or 5% of nucleic acids from any other size range or source.

According to the present invention, the nucleic acid sample useful for the methods of the present invention has been isolated from a cervical mucus sample from the host of a fetus, *e.g*., a pregnant woman. The cervical mucus sample of the present invention could have been obtained from the host of a fetus, at any time during the pregnancy, for example, during the first or second trimester, by any means now known or later discovered in the art. In general, a cervical mucus sample, *e.g* an endocervical mucus sample, can be obtained using techniques such as transcervical swabs, endocervical lavage, scrapes, cytobrush, aspiration, intrauterine lavage, or a combination thereof.

In one embodiment, the cervical mucus sample of the present invention is a fresh sample, *e.g.*, without substantial preservation or processing. In another embodiment, the cervical mucus sample is a sample preserved from a fresh sample, *e.g*., preserved in a suitable aqueous preservation or transportation medium, or alternatively, a sample of a medium containing nucleic acids leached from one or more cervical mucus samples. Without being bound to any theory, it is believed that nucleic acid will diffuse out from the cervical mucus into a fluid that is in contact with the mucus. Fetal nucleic acid will thus be present both in the cervical mucus sample as well as in the media in which the sample is stored and/or transported. Accordingly, the nucleic acid sample useful for the methods of the present invention can be obtained directly from the cervical mucus sample, or from the medium, for example, preservation medium, transportation medium, or any aqueous medium. that is in contact with the cervical mucus. Examples of transportation media include, but are not limited to, any tissue culture medium known to one of skill in the art, *e.g*., RPMI-1640 medium. In yet another embodiment, the cervical mucus sample of the present invention is maintained or stored between about 4°C and about 20°C, *e.g*., in a low calcium basal medium.

In still another embodiment, the cervical mucus sample of the present invention is a treated sample, *e.g*., a fresh sample or preserved sample treated with any suitable reagent(s) to facilitate mucous dissolution which in turn, assists in isolation of nucleic acid components from the sample. For example, the cervical mucus sample can be a sample treated with mucolytic agent(s) or mucinase(s), *e.g*., N-acetyl-L-cysteine, L-cysteine, dithiothreitol (DTT), bromhexine hydrochloride, and any of the hyaluronidases, including hyaluronate lyase, hyaluronoglucosarninidase, and hyaluronglucuronidase. In another example, the cervical mucus sample of the present invention is a sample treated with enzyme(s), *e.g*., sugar hydrolysis enzyme(s) such as β-galactosidase or invertase, or proteinase, or pepsin or combinations thereof. The cervical mucus sample may also be treated with chemicals known in the art to induce apoptosis to release fetal nucleic acid.

In another embodiment, the cervical mucus sample of the present invention is a sample treated to enrich fetal nucleic acid and/or reduce maternal nucleic acid content. For example, the cervical mucus sample can be treated to reduce or degrade any nucleic acid, *e.g*., DNA that is characteristic of maternal DNA. One of such nucleic acid is hypermethylated maternal DNA. Any means to reduce, degrade, or selectively remove hypermethylated maternal DNA can be used including, without any limitation, methylation specific restriction enzymes such as McrBC (BioLabs), antibodies specific for hypermethylated maternal DNA such as anti-5'-methyl-cytosine antibodies and/or anti-methylCpG binding protein-2 (MeCP2) antibodies, or ligands or proteins such as MeCP2 that specifically bind methylated CpG islands in maternal DNA.

Alternatively fetal nucleic acid can be enriched using markers specific for fetal nucleic acids. For example, hypomethylated maspin DNA can be used as a marker for fetal DNA. In one instance, one can treat total cervical mucous DNA with sodium bisulfite, which can induce chemical changes in the hypomethylated fetal DNA whereby unmethylated cytosine of fetal DNA is converted into uridine (U). Such change can be used to preferentially isolate or enrich fetal DNA, *e.g*., to preferentially amplify fetal DNA containing uridine(s) converted from cytosine(s).

According to the present invention, the nucleic acid sample of the present invention can be used to conduct genetic tests or screening of a fetus. In particular, the nucleic acid sample of the present invention can be used to test or screen the genetic composition of a fetus, *e.g*., chromosomal composition, gene composition, or genetic marker or finger printing pattern of a fetus. In one embodiment, testing or screening a genetic composition of a fetus includes probing for chromosomal abnormalities including, without any limitation, monosomy, partial monosomy, trisomy, partial trisomy, chromosomal translocation, chromosomal duplication, chromosomal deletion or microdeletion, and chromosomal inversion.

In general, the term "monosomy" refers to the presence of only one chromosome from a pair of chromosomes. Monosomy is a type of aneuploidy. Partial monosomy occurs when the long or short arm of a chromosome is missing. Common human genetic disorders arising from monosomy include: X0, only one X chromosome instead of the usual two (XX) seen in a normal female (also known as Turner syndrome); cri du chat syndrome, a partial monosomy caused by a deletion of the end of the short p (from the word petit, French for small) arm of chromosome 5; and 1p36 Deletion Syndrome, a partial monosomy caused by a deletion at the end of the short p arm of chromosome 1.

In contrast, the term "trisomy" refers to the presence of three, instead of the normal two, chromosomes of a particular numbered type in an organism. Thus the presence of an extra chromosome 21 is called trisomy 21. Most trisomies, like most other abnormalities in chromosome number, result in distinctive birth defects. Many trisomies result in miscarriage or death at an early age. A partial trisomy occurs when part of an extra chromosome is attached to one of the other chromosomes, or if one of the chromosomes has two copies of part of its chromosome. A mosaic trisomy is a condition where extra chromosomal material exists in only some of the organism's cells. While a trisomy can occur with any chromosome, few babies survive to birth with most trisomies. The most common types that survive without spontaneous abortion in humans include: Trisomy 21 (Down syndrome); Trisomy 18 (Edwards syndrome); Trisomy 13 (Patau syndrome); Trisomy 9; Trisomy 8 (Warkany syndrome 2); Trisomy 16 (which is the most common trisomy in humans, occurring in more than 1% of pregnancies. This condition, however, usually results in spontaneous miscarriage in the first trimester). Trisomy involving sex chromosomes include: XXX (Triple X syndrome); XXY (Klinefelter's syndrome); and XYY (XYY syndrome).

In another embodiment, testing or screening a genetic composition of a fetus includes probing for allele or gene abnormalities. *e.g*., one or more mutations such as point mutations, insertions, deletions in one or more genes.

In yet another embodiment, testing or screening a genetic composition of a fetus includes probing for one or more polymorphism patterns or genetic markers, *e.g*., short tandem repeat sequences (STRs), single nucleotide polymorphisms (SNPs), etc.

In still another embodiment, testing or screening a genetic composition of a fetus includes probing for any genetic abnormality corresponding to or associated with a condition or disorder, *e.g*., Cystic Fibrosis, Sickle-Cell Anemia, Phenylketonuria, Tay-Scahs Disease, Adrenal Hyperplasia, Fanconi Anemia, Spinal Muscularatrophy, Duchenne's Muscular Dystrophy, Huntington's Disease, Beta Thalassaemia, Myotonic Dystrophy, Fragile-X Syndrome, Down Syndrome, Edwards Syndrome, Patau Syndrome, Klinefelter's Syndrome. Triple X syndrome, XYY syndrome, Trisomy 8, Trisomy 16, Turner Syndrome, Robertsonian translocation, Angelman syndrome, DiGeorge Syndrome, Wolf-Hirschhorn Syndrome, RhD Syndrome, Tuberous Sclerosis, Ataxia Telangieltasia, and Prader-Willi syndrome..

In still another embodiment, testing or screening a genetic composition of a fetus includes probing for any genetic abnormality that is not uniquely associated with Y chromosome.

In still another embodiment, testing or screening a genetic composition of a fetus includes probing for any genetic condition corresponding to or associated with gender or paternity of the fetus.

Usually genetic tests provided by the present invention use the nucleic acid sample of the present invention either directly or as templates for "amplification-based" genetic composition testing assays, including without any limitation, polymerase chain reaction ("PCR"), real-time polymerase chain reaction ("RT-PCR"), ligase chain reaction ("LCR"), self-sustained sequence replication ("3SR") also known as nucleic acid sequence based amplification ("NASBA"). Q-B-Replicase amplification, rolling circle amplification ("RCA"), transcription mediated amplification ("TMA"), linker-aided DNA amplification ("LADA"), multiple displacement amplification ("MDA"), invader and strand displacement amplification ("SDA"). Amplification of a nucleotide fragment using a pair of primers specific for an allele indicates the presence of the allele.

In one embodiment, the "amplification-based" genetic composition testing assays of the present invention include using primers to generate amplicons less than about 200 base pairs, less than about 150 base pairs, or between about 75 to about 150 base pairs. Exemplary primers of the invention used in the amplification-based assays are provided herein. In one embodiment, the primers of the invention include, but are not limited to, the pairs of primers of SEQ ID NOs: 1 and 2; SEQ ID NOs: 3 and 4; SEQ ID NOs: 5 and 6; SEQ ID NOs: 9 and 10; SEQ ID NOs: 11 and 12; and SEQ ID NOs: 13 and 14. In another embodiment, exemplary primers of the invention include, but are not limited to, the primer sets listed in Tables 2, 3, 4 and 5.

According to another aspect of the present invention, it provides a method of isolating a fetal nucleic acid sample. The method comprises isolating, by size fractionation, one or more nucleic acid samples from a cervical mucus sample obtained from a maternal host of a fetus in a size range enriched with fetal nucleic acids. Examples of such size range include without any limitation from about 50 to about 300 base pairs, from about 50 to about 250 base pairs, from about 50 to about 200 base pairs, from about 50 to about 150 base pairs, or from about 50 to about 100 base pairs or a combination thereof. In one embodiment, the nucleic acid sample does not contain a substantial amount, *e.g*., more than 0.5%, 1%, 2%, 3%, 4%, or 5% of nucleic acids from any other size range or source.

The disclosure also provides an isolated nucleic acid sample useful for genetic testing of a fetus. The nucleic acid sample, *e.g*., a DNA sample, can be obtained by isolating nucleic acid fragments of from about 50 base pairs to about 100, 200, 300, 400, 500, or 1000 base pairs in length from a cervical mucus sample obtained from a female subject containing the fetus. In one embodiment, these nucleic acid fragments are obtained from the total nucleic acid isolated from the cervical mucus sample by a size fractionation method. In another embodiment, the isolated nucleic acid is substantially free of non-nucleic acid components.

The disclosure also provides kits useful for genetic testing or screening of a fetus. In one embodiment, the kit contains one or more pairs of primers useful for genetic composition testing assays and optionally one or more probes useful for detecting the amplified product(s) by the primers. In another embodiment, the kit contains one or more pairs of primers useful for testing one or more polymorphisms or genetic markers of a fetus. In yet another embodiment, the kit contains one or more pairs of primers which are useful for generating amplicons less than about 200 base pairs, less than about 150 base pairs, or between about 75 to about 150 base pairs. In still another embodiment, the kit contains one or more pairs of primers for one or more designated chromosomes and the primers are selected from the primer sets listed in Tables 2, 3, 4 or 5. In a further embodiment, the kit contains the pairs of primers of SEQ ID NOs: t and 2; SEQ ID NOs: 3 and 4; SEQ ID NOs: 5 and 6; SEQ ID NOs: 9 and 10; SEQ ID NOs: 11 and 12; SEQ ID NOs: 13 and 14; or a combination thereof. The kit can also optionally contain one or more probes and/or other suitable reagents useful for detecting the amplified product(s) by the primers. Further, the kit can comprise instructions for using the pair of primers to test the genetic composition of a fetus.

In still another embodiment, the kit contains one or more nucleic acid samples of the present disclosure. In a further embodiment, the kit contains the cervical mucus sample of the present disclosure and an instruction for isolating the nucleic acid sample of the present disclosure from the cervical mucus sample.

### EXAMPLES

The following examples are intended to illustrate but not to limit the invention in any manner, shape, or form, either explicitly or implicitly. While they are typical of those that might be used, other procedures, methodologies, or techniques known to those skilled in the art may alternatively be used.

### Example 1

This Example describes the collection and isolation of fetal DNA from pregnant women.

Cervical mucous samples were collected from patients, after due consent, by cytobrush method. In the cytobrush method, a Pap smear cytobrush (*e.g*., MedScand-AB, Malmo, Sweden) was inserted to a maximum depth of 2 cm and removed while rotating it a full turn (*i.e*., 360°). In order to remove the transcervical cells caught on the brush, the brush was shaken into a test tube containing 2-3 ml of a tissue culture medium (*e.g*., RPM1-1640 medium, available ATCC, Virginia) in the presence of 1% Penicillin Streptomycin antibiotic. In order to concentrate the transcervical cells on microscopic slides cytospin slides were prepared using *e.g*., a Cytofunnel Chamber Cytocentrifuge (Thermo-Shandon, England). The conditions used for cytocentrifugation are dependent on the murkiness of the transcervical specimen; if the specimen contained only a few cells, the cells are first centrifuged for five minutes and then suspended with 1ml of fresh medium. Once prepared, the cytospin slides can be kept in 95% alcohol until further use.

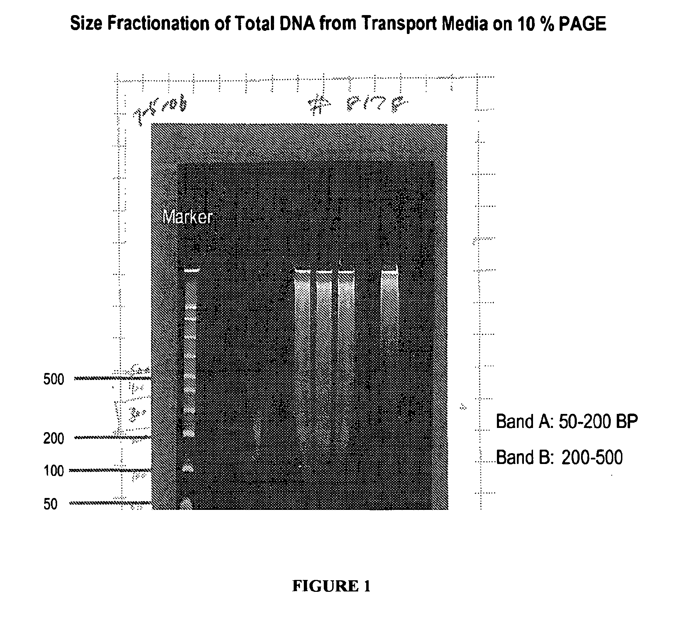

DNA was extracted from fetal tissues, mucous samples or the transport media using Roche's Apoptotic DNA-Ladder Kit following manufacturer's protocol with slight modification. Mucous samples were incubated with equal volume of lysis buffer for 30 minutes to 2 hours or until all the mucous had been dissolved. Some samples needed to be homogenized with a 21 gauze 1.5 inch long needle to facilitate complete mucous dissolution. Total mucous DNA was then size fractionated on 10% PAGE, also known as 10% TBE gel (Invitrogen) under non-denaturing conditions, and the small, 100-250 base pair long DNA band (see Figure 1) was sliced out after staining the gel with SYBR Gold stain. Fetal DNA from the gel was extracted by soaking the crushed gel in 0.3M sodium acetate (pH 5.5) at 37°C for overnight followed by desalting the DNA using Promega's Wizard SV Genomic DNA Purification kit.

### Example 2

This Example demonstrates that the DNA obtained from the cervical mucous samples after PAGE purification is indeed fetal DNA.

The total DNA obtained from the cervical swap was size fractionated on 10% PAGE, and the small, 50-250 base pair DNA band (see Figure 1) was sliced out. The DNA was extracted from PAGE using Promega's Membrane Binding buffer, and its concentration was determined by NanoDrop-1000 Spectrophotometer.

10-20 ng of this size-fractionated DNA was amplified by PCR with primers designed to amplify short STR regions (*e.g*., D22S1045, CSF1PO, D2S441 see Table 1 for detail).

Typical PCR reaction components were:

| | |
|---|---|
| 10 mM dNTP | 2.0 µl |
| 25 mM MgCl2 | 1.5 µl |
| 50 mM Primers | 0.5 µl |
| Template 1 µg/µl | 2.0 µl |
| Ampli Taq Gold | 0.5 µl |
| 10X PCR Buffer | 2.5 µl |
| Water | 16.0 µl |

Typical PCR cycle consisted of: Denaturation temperature of 94 °C for 30 sec. annealing temperature varied from 56 to 62°C depending upon the primer length, extension was done at 72 °C. Number of cycles used ranged from 26 to 40.

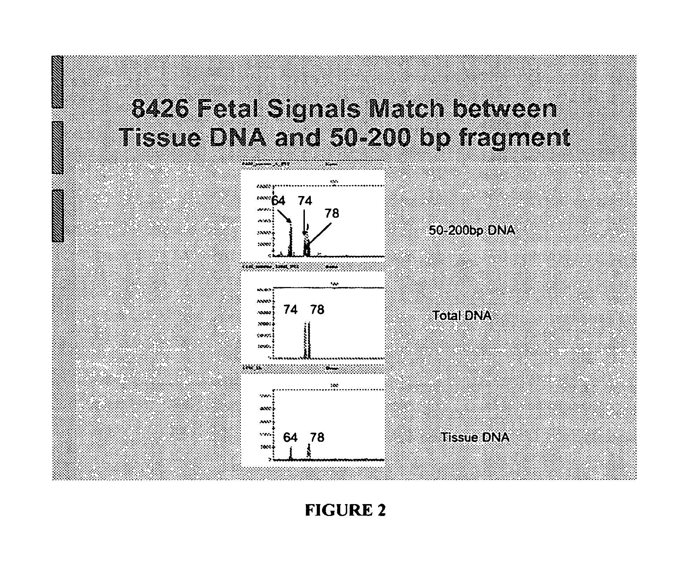

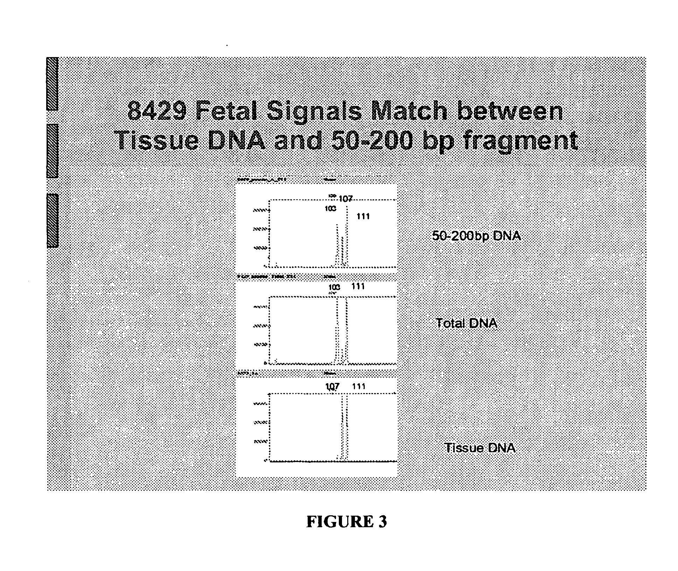

These primers were also used for PCR reaction with the DNA extracted from fetal tissues and the total, unfractionated, mucous DNA. Shown in FIG. 2 and FIG. 3 are PCR electropherograms that demonstrating that the 50-200 base pair DNA fraction resulted in the same fetal alleles as seen in fetal tissue PCR.

### Example 3

This Example demonstrates that the mini-STR markers detect fetal alleles.

Mini-STR markers of the invention were used to detect fetal alleles from DNA extracted from clinical cervical mucous samples. Table 1, below, summarizes the results obtained. D1S1677-F and -R, D22S 1045-F and -R, D10S1248-F and -R, TPOX, Mini-LFG33-F and -R, and Mini-LFG34-F and -R are exemplary primers of the invention.

**Table 1: Detection of Fetal Allele from Clinical Cervical Mucus Samples**

| **Sample ID** | **Sample Type** | **Gel Enriched** | **PCR Analysis** | | | | | **Presence of Fetal Allele** |
|---|---|---|---|---|---|---|---|---|
| | | | **Informative Primer Set** | **Sequence** | **Seq. ID No.** | **Maternal Alleles** | **Fetal Allele** | |
| 7601 | Transport Media | | D1S1677-F | FAM-TTCTGTTGGTATAGAGCAGTGTTT | SEQ ID NO: 1 | 90.21, 94.91 | 99.91 | Yes |
| | | | D1S1677-R | GTGACAGGAAGGACGAATG | SEQ ID NO: 2 | | | |
| 7602 | Transport Media | | D22S1045-F | FAM-ATTTTCCCCGATGATAGTAGTCT | SEQ ID NO: 3 | 95.11, 97.81 | 85.61 | Yes |
| | | | D22S1045-R | GCGAATGTATGATTGGCAATATTTTT | SEQ ID NO: 4 | | | |
| 7604 | Transport Media | | 022SIO45-F | FAM-ATTTTCCCCGATGATAGTAGTCT | SEQ ID NO: 3 | 98.15 | 92.44 | Yes |
| | | | D22S1045-R | GCGAATGTATGATTGGCAATATTTTT | SEQ ID NO: 4 | 102.31 | | |
| | | 8% PAGE | D10S1248-F | FAM-TTAATGAATTGAACAAATGAGTGAG | SEQ ID NO: 5 | 104.21 | 10.6.55 | |
| | | | D10S1248-R | GCAACTCTGGTTGTATTGTCTTCAT | SEQ ID NO: 6 | 111.12 | | |
| 7843 | Transport Media | | D10S1240-F | TAMRA-TCACCTGTCCCAGCTATCTG | SEQ ID NO: 7 | 101.33 | 104.66 | Yes |
| | | | D10S1240-R | AAAATTACTGGTACACTTGATAGCT | SEQ ID NO: 8 | 107.54 | | |
| 7845 | Transport Media | | D1S1677-F | FAM-TTCTGTTGGTATAGAGCAGTGTTT | SEQ ID NO: 1 | 97.11 | 105.71 | Yes |
| | | | D1S1677-R | GTGACAGGAAGGACGAATG | SEG ID NO: 2 | 101.91 | | |
| 7846 | Transport Media | | D1S1677-F | FAM-TTCTGTTGGTATAGAGCAGTGTTT | SEQ ID NO: 1 | 93.67 | 46.22 | Yes |
| | | | D1S1677-R | GTGACAGGAAGGACGAATG | SEQ ID NO: 2 | 102.33 | | |
| 8034 | Media | | TPOX-F | NED-CTTAGGGAACCCTCACTGAATG | SEQ ID NO 9 | 148.55 | 160.32 | Yes |
| | | | TPOX-R | GTCCTTGTCAGCGTTTATTTGC | SEQ ID NO: 10 | 156.34 | | |
| 8379 | Mucus | 10% PAGE | Profiler-F | commercially available from ABI | | 172.1, 176 | 125, 245 | Yes |
| | | | Profiler-R | commercially available from ABI | | | | |
| 9558 | Mucous Muccus | 1.5% Agarose | Mini-LFG34-F | FAM-CACAAGGCAGAATAAAGGGA | SEQ ID NO: 11 | 134, 150 | 142 | Yes |
| | | | Mini-LFG34-R | TTCATAGTCTGTCTTGTCTTGTCTCA | SEQ ID NO: 12 | | | |
| 9560 | Mucous | 1.5 % Agarose | Mini-LFG33-F | TAMRA-CACCATACCCAGCCTTACTG | SEQ ID NO. 13 | 118, 122 | 116 | Yes |
| | | | Mini-LFG33-R | CATGTTACTGTGCTGAATATTGTAGGC | SEQ ID NO: 14 | | | |
| 9561 | Mucous Muccus | 1.5% Agarose | Mini-LFG34-F | FAM-CACAAGGCAGAATAAAGGGA | SEQ ID NO: 13 | 135, 150 | 146 | Yes |
| | | | Mini-LFG34-R | TTCATAGTCTGTCTTGTCTTGTCTCA | SEQ ID NO: 14 | | | |

**Table 2: Mini-STR Primers for Chromosome 21**

| Note: * = *6-FAM. VIC, NED, JOE, ROX, PET*, *TAMRA or 5-FAM* | | | | | | |
|---|---|---|---|---|---|---|
| **Number** | **Name/Loci** | **5' Label** | **Sequence** | **Seq. ID No.** | **Length** | **Chromosome** |
| p21M-1 | Mini LFG20-F | 6-FAM | TTGAGAAGGCCCCACCTATG | SEQ ID NO: 15 | 20 | 21 |
| p21M-2 | Mini LFG20-R | | AGAAGGCCCCCTGTGTTATG | SEQ ID NO: 16 | 20 | 21 |
| p21M-3 | Mini LFG21-F | HEX | GCGAATCATGACACTAATTTTGG | SEQ ID NO: 17 | 23 | 21 |
| p21M-4 | Mini LFG21-R | | TGGAGAAGAAAAAGAGGCCTGA | SEQ ID NO: 18 | 22 | 21 |
| p21M-5 | Mini LFG24-F | NED | GGTTCMGGAAAATTGTTTAGGC | SEQ ID NO: 19 | 24 | 21 |
| p21M-6 | Mini LFG24-R | | TGCTCTGGACTTACAGCATCAA | SEQ ID NO: 20 | 22 | 21 |
| p21M-7 | Mini LFG26-F | 6-FAM | CCCTTAAAACCATATTTTTCACCTC | SEQ ID NO: 21 | 25 | 21 |
| p21M-8 | Mini LFG26-R | | AGCCTGGGTGACAGAGCAAG | SEQ ID NO: 22 | 20 | 21 |
| p21M-9 | Mini LFG29-F | HEX | TTGCTTGAGAGGTAAAAAGAAAA | SEQ ID NO: 23 | 23 | 21 |
| p21M-10 | Mini LFG29-R | | GAGCAACAGAGCGAGATTCTG | SEQ ID NO: 24 | 21 | 21 |
| p21 M-11 | Mini LFG33-F | NED | CACCATACCCAGCCTTACTG | SEQ ID NO: 25 | 20 | 21 |
| p21M-12 | Mini LFG33-R | | CATGTTACTGTGCTGAATATTGTAGGC | SEQ ID NO: 26 | 27 | 21 |
| p21M-13 | Mini LFG34-F | 6-FAM | GGCAGAATAAAGGGATTATTGC | SEQ ID NO: 27 | 22 | 21 |
| p21M-14 | Mini LFG34-R | | TTCATAGTCTGTCTTGTCTTGTCTCA | SEQ ID NO: 28 | 26 | 21 |
| p21M-15 | Mini D21S2054-F | NED | GCAGTAAATGTCTATGAAACAAGG | SEQ ID NO: 29 | 24 | 21 |
| p21M-16 | Mini D21S2054-R | | TGATAAATAGTGAATATAGTTGACAGC | SEQ ID NO: 30 | 27 | 21 |
| p21M-17 | Mini D21S1904-F | * | CAACAATTCCTTCTAATTTTCCA | SEQ ID NO: 31 | 23 | 21 |
| p21M-18 | Mini D21S1904-R | | TGTCTGGTTTCCCCATCTCT | SEQ ID NO: 32 | 20 | 21 |
| p21M-19 | Mini D21S1911-F | * | TGAGGAGACATCCTTGACAAAA | SEQ ID NO: 33 | 22 | 21 |
| p21M-20 | Mini D21S1911-R | | CATACACACAGCAAGTATGAGTGA | SEQ ID NO: 34 | 24 | 21 |
| p21 M-21 | Mini D21S1256-F | * | GCCTATGGTCCCATCATAACA | SEQ ID NO: 35 | 21 | 21 |
| p21M-22 | Mini D21S1256-R | | TCCACAGTTCTTAGATGGCTTT | SEQ ID NO: 36 | 22 | 21 |
| p21M-23 | Mini D21S1899-F | * | TGAAAACGTGTTGACAGATGAA | SEQ ID NO: 37 | 22 | 21 |
| p21M-24 | Mini D21S1899-R | | AATGGCAGGATTTTCTTTTT | SEQ ID NO: 38 | 20 | 21 |
| p21M-25 | Mini D21S1922-F | * | TGTCAAAATATGTGGATTAGACAAAA | SEQ ID NO: 39 | 26 | 21 |
| p21M-26 | Mini D21S1922-R | | TCACTGGTATACTGTGATGTGTGC | SEQ ID NO: 40 | 24 | 21 |
| p21M-27 | Mini D21S1884-F | * | AAAAATTATTGATAACGTTCAGTAT | SEQ ID NO: 41 | 25 | 21 |
| p21M-28 | Mini D21S1884-R | | TTTCTAACAATATGTACCCACTGGA | SEQ ID NO: 42 | 25 | 21 |
| p21 M-29 : | Mini D21S1914-F | * | CATTGGGCCTTCTGTCAAAT | SEQ ID NO: 43 | 20 | 21 |
| p21M-30 | Mini D21S1914-R | | TCTGCAGAATTTCATTTGCTGT | SEQ ID NO: 44 | 22 | 21 |
| p21M-31 | Mini D21S263-F | * | CAAACTTGAAATATGAAAAAGTCATCA | SEQ ID NO: 45 | 27 | 21 |
| p21M-32 | Mini D21S263-R | | TTTCTGTATTTCCTGAAACAACATTT | SEQ ID NO: 46 | 26 | 21 |
| p21M-33 | Mini D21S1252-F | | TCTGTCTTTGTCTCACTATCTGTCTG | SEQ ID NO: 47 | 26 | 21 |
| p21M-34 | Mini D21S1252-R | | TAGGGTGAGGACCCCTTTCT | SEQ ID NO: 48 | 20 | 21 |
| p21M-35 | Mini D21S1919-F | * | CCTGGATTATTTGTTCAAAGTCAG | SEQ ID NO: 49 | 24 | 21 |
| p21M-36 | Mini D21S1919-R | | TCTCATGTTCCTTGGCCTGT | SEQ ID NO: 50 | 20 | 21 |
| p21M-37 | Mini D21S1255-F | * | ATTTTGCCACATAGAGAAAAATA | SEQ ID NO: 51 | 23 | 21 |
| p21M-38 | Mini D21S1255-R | | * GCCTGGACATCCTCTTTCT | SEQ ID NO: 52 | 19 | 21 |
| p21M-39 | Mini D21S266-F | * | AGATGTAGCACAGTTAGATGCAGA | SEQ ID NO: 53 | 24 | 21 |
| p2 1 M-40 | Mini D21S266-R | | AGCAGAAAAAGCCATTCTGG | SEQ ID NO: 54 | 20 | 21 |
| p21M-41 | Mini D21S2058-F | * | GTCATGACCCTGGCTGTG | SEQ ID NO: 55 | 18 | 21 |
| p21M-42 | Mini D21S2058-R | | AGGGCAGGCTGTGCTCAT | SEQ ID NO: 56 | 18 | 21 |
| p21M-43 | Mini D21S1431-F | * | GGGACCATTTTAGATATTCTGCT | SEQ ID NO: 57 | 23 | 21 |
| p21M-44 | Mini D21S1431-R | | GCACTTAACAAGCACTGAATCAA | SEQ ID NO: 58 | 23 | 21 |
| p21M-45 | Mini D21S259-F | * | TCCTGAAGGAAGAATGTGGTC | SEQ ID NO: 59 | 21 | 21 |
| p21M-46 | Mini D215259-R | | ATGCATGGTCGTGTGTGTG | SEQ ID NO: 60 | 19 | 21 |
| p21M-47 | Mini D21S270-F | * | TTTTTCAAAATCAAAAAGATAGTGA | SEQ ID NO: 61 | 25 | 21 |
| p21M-48 | Mini D21S270-R | | GGAGGGCATCTGGGTAATTT | SEQ ID NO: 62 | 20 | 21 |
| p21M-49 | Mini D21S1912-F | * | GCCATCAGCCCTCATACAGA | SEQ ID NO: 63 | 20 | 21 |
| p21M-50 | Mini D21S1912-R | | GAATTTGGGGGACGCAGT | SEQ ID NO: 64 | 18 | 21 |
| p21M-51 | Mini D21S260-F | * | CGAGAAGTTTCCCATGCATTT | SEQ ID NO: 65 | 21 | 21 |
| p21M-52 | Mini D21S260-R | | AAATTCAGTGATGGGAAGAAGG | SEQ ID NO: 66 | 22 | 21 |
| p21M-53 | Mini D21S261-F | * | CCTAAAACAGCATCAACAGAAA | SEQ ID NO: 67 | 22 | 21 |
| p21M-54 | Mini D21S261-R | | TTGGACCTTTTGATTTTTCCT | SEQ ID NO: 68 | 21 | 21 |
| p21 M-55 | Mini D21S262-F | * | CAGCAACTCCCACTTCTGAC | SEQ ID NO: 69 | 20 | 21 |
| p21M-56 | Mini D21S262-R | | TTGTTGTTGAGTGAAAGAATAGAGAAA | SEQ ID NO: 70 | 27 | 21 |
| p21M-57 | Mini D21S1892-F | * | AAATCTGAATTATGTCCAATCAAAA | SEQ ID NO: 71 | 25 | 21 |
| p21M-58 | Mini D21S1892-R | | TGAGTTTTTGGAAGAGAGAGAGAGA | SEQ ID NO: 72 | 25 | 21 |
| p21M-59 | Mini D21S272-F | * | AAAAGGGGATCCAATATGAAA | SEQ ID NO: 73 | 21 | 21 |
| p21M-60 | Mini D21S272-R | | TGGAACAATTTTATCCTTAGTTTGTC | SEQ ID NO: 74 | 26 | 21 |
| p21M-61 | Mini D21S1893-F | * | TATGCACACCACACGGACAC | SEQ ID NO: 75 | 20 | 21 |
| p21M-62 | Mini D21S1893-R | | GTTCCGGGGAAGTTTTATGC | SEQ ID NO: 76 | 20 | 21 |
| p21M-63 | Mini D21S265-F | * | TGGCAAAGAGAACAACAGCA | SEQ ID NO: 77 | 20 | 21 |
| p21M-64 | Mini D21S265-R | | TTCTGTGAATATGGGTTCTGGA | SEQ ID NO: 78 | 22 | 21 |
| p21 M-65 | Mini D21S267-F | * | GGGGATTATTTATGTAGAAAATGAGA | SEQ ID NO: 79 | 26 | 21 |
| p21M-66 | Mini D21S267-R | | GGTGACAGACCCTGTCTCTAAAA | SEQ ID NO: 80 | 23 | 21 |
| p21M-67 | Mini D21S268-F | * | TGGGCAACAGAGTGAGACAG | SEQ ID NO: 81 | 20 | 21 |
| p21M-68 | Mini D21S268-R | | CACATCCTTGCCAACACTTG | SEQ ID NO: 82 | 20 | 21 |
| p21M-69 | Mini D21S269-F | * | GATACTGAATCATCCCTTTCATTC | SEQ ID NO: 83 | 24 | 21 |
| p21M-70 | Mini D21S269-R | | TTCCGTTATTAATTTTATTCTGAGG | SEQ ID NO: 84 | 25 | 21 |
| p21M-71 | Mini D21S1902-F | * | GAGTGAGAGACAGACAGAGAGACG | SEQ ID NO: 85 | 24 | 21 |
| p21M-72 | Mini D21S1902-R | | CAGTAGGDGCAGACTATTTTACTC | SEQ ID NO:86 | 24 | 21 |
| p21M-73 | Mini D21S1253-F | * | ATGGGTGACAGAGCGAGACT | SEQ ID NO: 87 | 20 | 21 |
| p21M-744 | Mini D21S1253-R | | TTCAGAGCCTGGGTTAAACA | SEQ ID NO: 88 | 20 | 21 |
| p21M-75 | Mini D21S1254-F | * | AATGACCATCCTTAAAACAACTTT | SEQ ID NO: 89 | 24 | 21 |
| p21M-76 | Mini D21S1254-R | * | GTGGCTGAGCGAGACTCTGT | SEQ ID NO: 90 | 20 | 21 |
| p21M-77 | Mini D21S1907-F | * | TCACTCAATTTATGAGAGCGAAA | SEQ ID NO: 91 | 23 | 21 |
| p21M-78 | Mini D21S1907-R | | TCAGCCTTTGATATGTGCATT | SEQ ID NO: 92 | 21 | 21 |
| p21M-79 | Mini D21S1909-F | * | GCATGAGTGGAAAAATGTGAAA | SEQ ID NO: 93 | 22 | 21 |
| p21M-80 | Mini D21S1909-R | | CTGAGTCAAGAGCAGGCAACT | SEQ ID NO: 94 | 21 | 21 |
| p21M-81 | Mini D21S1910-F | * | CCAATGCTTTTGATTTTTAAGC | SEQ ID NO: 95 | 22 | 21 |
| p21M-82 | Mini D21S1910-R | | CGGCAAAGTAGTATTTAATGTGCT | SEQ ID NO: 96 | 24 | 21 |
| p21M-83 | Mini D21S1257-F | * | TTTCATTCACCGGTTTTCCT | SEQ ID NO: 97 | 20 | 21 |
| p21M-84 | Mini D21S1257-R | | TTTTAGGGTATATCTGCCATAATGC | SEQ ID NO: 98 | 25 | 21 |
| p21M-85 | Mini D21S1258-F | * | GGGAAGGAAAATAAAGCATTGA | SEQ ID NO: 99 | 22 | 21 |
| p21 M-86 | Mini D21S1258-R | | GCACAAAAACAAAATCTGTCACT | SEQ ID NO: 100 | 23 | 21 |
| p21M-87 | Mini D21S1913-F | * | TTGCTGGGTTGTTAAACTTATTCA | SEQ ID NO: 101 | 24 | 21 |
| p21M-88 | Mini D21S1913-R | | AAAGGTGAACGCTGGTATCG | SEQ ID NO: 102 | 20 | 21 |
| p21M-89 | Mini D21S1259-F | * | GACCCCAACACTGGACACA | SEQ ID NO: 103 | 19 | 21 |
| p21M-90 | Mini D21S1259-R | | CTTGGTAAGTGGGCAGTGAG | SEQ ID NO: 104 | 20 | 21 |
| p21M-91 | Mini D21S1915-F | * | CATGCTCATAGATATGCACACA | SEQ ID NO: 105 | 22 | 21 |
| p21M-92 | Mini D21S1915-R | | GATTGTGCCAGGTCTCAGGT | SEQ ID NO: 106 | 20 | 21 |
| p21M-93 | Mini D21S1916-F | * | CCAAGGTGAATTCCCAATTT | SEQ ID NO: 107 | 20 | 21 |
| p21M-94 | Mini D21S1916-R | | GCGGCACATTTTCACAGACT | SEQ ID NO: 108 | 20 | 21 |
| p21M-95 | Mini D21S1917-F | * | TGAACATTAACACTGGTAACTTTACAT | SEQ ID NO: 109 | 27 | 21 |
| p2lM-96 | Mini D21S1917-R | | TGTCCTCTCCATTTTGCTTG | SEQ ID NO: 110 | 20 | 21 |
| p21M-97 | Mini D21S1918-F | | CTTCTCTCCAATTCCCATGC | SEQ ID NO: 111 | 20 | 21 |
| p21M-98 | Mini D21S1918-R | | GAAGGAAAAATTGGGATTTCG | SEQ ID NO: 112 | 21 | 21 |
| p21M-99 | Mini D21S1920-F | * | CAGCCTGGGTGACAGAGAC | SEQ ID NO: 113 | 19 | 21 |
| p21M-100 | Mini D21S1920-R | | TGTTGATGAAGCATTTACTCATACAT | SEQ ID NO: 114 | 26 | 21 |
| p21M-101 | Mini D21S1921-F | * | TCCCCCTAAATGGACAACTTT | SEQ ID NO: 115 | 21 | 21 |
| p21M-102 | Mini D21S1921-R | | GCTTTGTTTTCCTTTAGCTTCC | SEQ ID NO: 116 | 22 | 21 |
| p21M-103 | Mini D2151883-F | * | TCTGGAATGGTTAAGGCAGAA | SEQ ID NO: 117 | 21 | 21 |
| p21M-104 | Mini D21S1883-R | | CACCATTCTCCCTAGCATGA | SEQ ID NO: 118 | 20 | 21 |
| p21M-105 | Mini D21S1885-F | | CAAGGTGGAAGGCAGAAGG | SEQ ID NO: 119 | 19 | 21 |
| p21M-106 | Mini D21S1885-R | | CGCGCTCTCTCTCTCTCTCT | SEQ ID NO: 120 | 20 | 21 |
| p21M-107 | Mini D21S1886-F | | TGCAAGATTTCCCCCTTCTA | SEQ ID NO: 121 | 20 | 21 |
| p21M-108 | Mini D21S1886-R | | CTTTGACTCCTCAGCCGTGT | SEQ ID NO: 122 | 20 | 21 |
| p21M-109 | Mini D21S1887-F | | CCTGGCATCTCTGTTTTA | SEQ ID NO: 123 | 19 | 21 |
| p21M-110 | Mini D21S1887-R | | AAAGATGATGTCAGGAATGC | SEQ ID NO: 124 | 20 | 21 |
| p21m-111 | Mini D21S1260-F | * | CATCCAAGGGGAACACAAGT | SEQ ID NO:125 | 20 | 21 |
| p21M-112 | Mini D2151260-R | | GGAAGTAAAAGGCCCAGAGG | SEQ ID NO: 126 | 20 | 21 |
| p21M-113 | Mini D21S1888-F | * | AAAMGGATGTTTGTTATGGTAAGA | SEQ ID NO:127 | 25 | 21 |
| p21M-114 | Mini D21S1888-R | | GAACTGTGTGCCAGGAACTG | SEQ ID NO: 128 | 20 | 21 |
| p21M-15 | Mini D21S1889-F | * | TGTGTGTTTGCATGTATGTGTAGA | SEQ NO: 129 | 24 | 21 |
| p21M-116 | Mini D21S1889-R | | ACAGAAACATGGCTGCCTCT | SEQ ID NO: 130 | 20 | 21 |
| p21 M-111 | Mini D21S1890-F | * | GACCACAGATTTCCCAATCG | SEQ ID NO: 131 | 20 | 21 |
| p21M-118 | Mini D21S1890-R | | AAACCAACTGACTCCCAAACA | SEQ ID NO: 132 | 21 | 21 |
| p21M-119 | Mini D21S1891-F | * | CAGAGCGAGACTCCATCTCA | SEQ ID NO: 133 | 20 | 21 |
| p21M-120 | Mini D21S1891-R | | GGAACCCTTGGATGTTGCTA | SEQ ID NO: 134 | 20 | 21 |
| p21M-121 | Mini D21S1894-F | * | GCTCAATGCTATTGGAGTGC | SEQ ID NO: 135 | 20 | 21 |
| p21M-122 | Mini D21S1894-R | | TTCACAAAACAGAACCAACCA | SEQ ID NO: 136 | 21 | 21 |
| p21M-1223 | Mini D21S1895-F | * | TCTCCCTTGCCAGACTTCTC | SEQ ID NO: 137 | 20 | 21 |
| p21M-124 | Mini D2151895-R | | GCCCAGGATGGACAAAGA | SEQ ID NO: 138 | 18 | 21 |
| p21M-125 | Mini D2151896-F | | CACATGTGGCCACTGCAC | SEQ ID NO: 139 | 18 | 21 |
| p21M-126 | Mini D21S1896-R | | CCAGGGATTTGTCTAGAAAGGA | SEQ ID NO: 140 | 22 | 21 |
| p21M-127 | Mini D21S1897-F | * | GCCTAGGCAACCAGAGTGAG | SEQ ID NO: 141 | 20 | 21 |
| p21M-128 | Mini D21S1897-R | | TGTATTTCTTTTCTTTTTAAATGGTGA | SEQ ID NO: 142 | 27 | 21 |
| p21M-129 | Mini D21S1898-F | * | CTCTTCAGCAGCCGAGAAAA | SEQ ID NO: 143 | 20 | 21 |
| p21M-130 | Mini D21S1898-R | | CACCAGAAAGCAAGGAAGGA | SEQ ID NO: 144 | 20 | 21 |
| p21M-131 | Mini D21S1900-F | * | ACACCAGAGGGCAGAGTGAG | SEQ ID NO: 145 | 20 | 21 |
| p21M-132 | Mini D2151900-R | | AAAGCACTGTATGTTTCTGTGAGA | I SEQ ID NO: 146 | 24 | 21 |
| p21M-133 | Mini D21S1901-F | * | GGTTGTCCAGAGAAACAACCA | SEQ ID NO: 147 | 21 | 21 |
| p21M-134 | Mini D21S1901-R | | CAATTGTGTGAGCCAATCTCTC | SEQ ID NO: 148 | 22 | 21 |
| p21M-135 | Mini D21S1903-F | * | GGCAATTCCTTAMATAAATCACTC | SEQ ID NO: 149 | 25 | 21 |
| p21M-136 | Mini D21S1903-R | * | GCCCGGCCCTATCTATCTAT | SEQ ID NO: 150 | 20 | 21 |
| p21M-137 | Mini D21S1905-F | | GCATGCTCGCTCTCTCTCTT | SEQ ID NO: 151 | 20 | 21 |
| p21M-138 : | Mini D21S1905-R | | AACTGGCGTGTCTACACCATC | SEQ ID NO: 152 | 21 | 21 |
| p21M-139 | Mini D21S1906-F | * | GAGCAAGACTCCATCTCAAAAA | SEQ ID NO: 153 | 22 | 21 |
| p21M-140 | Mini D21S1906-R | | AAAGATTGCCCAACAAATGG | SEQ ID NO: 154 | 20 | 21 |
| p21M-141 | Mini D21S1908-F | * | TGGGTTCCATAGTTCTAATGTGTG | SEQ ID NO: 155 | 24 | 21 |
| p21 M-142 | Mini D21S1908-R | | TCTCTGGCTGGACATACTATTCA | SEQ ID NO: 156 | 23 | 21 |
| p21M-143 | Mini D21S1438-F | * | GGAGAAGGTAGCTAAAGGATGAAA | SEQ ID NO: 157 | 24 | 21 |
| p21M-144 | Mini D21S1438-R | | TGCACCCTAGGACCTAAAGAA | SEQ ID NO: 158 | 21 | 21 |
| p21M-145 | Mini D21S1439-F | * | CCTGATCTGGTCCTGTAGCC | SEQ ID NO: 159 | 20 | 21 |
| p21M-146 | Mini D21S1439-R | | TGAGMGAAAATAAAGTGTTCTGC | SEQ ID NO: 160 | 25 | 21 |
| p21M-147 | MiniATA42C09-F | * | CAAAGCGAGACCTTTTCTCAA | SEQ ID NO: 161 | 21 | 21 |
| p21M-148 | Mini ATA42C09-R | | CGAGTCATAGATCCATTACCCATT | SEQ ID NO: 162 | 24 | 21 |
| p21M-149 | Mini D21S1441-F | * | ACAACCGAGCGAGACCTG | SEQ ID NO: 163 | 18 | 21 |
| p21M-150 | Mini D21S1441-R | | GGAACTGATGGTCACAAGATAGTC | SEQ 10 NO: 164 | 24 | 21 |
| p2lM-151 | Mini D21S120-F | * | TTGTGGATTTTCCCAATTGAT | SEQ ID NO: 165 | 21 | 21 |
| p21M-152 | Mini D21S120-R | | TTTGTATTTGCCTAAAAACAGAGC | SEQ ID NO: 166 | 24 | 21 |
| p21M-153 | Mini D21S167-F | * | TACCAGCTTCAAACGTGCAG | SEQ ID NO:167 | 20 | 21 |
| p21M-154 | Mini D21S167-R | | CCTTGCCCTGAAGCACAT | SEQ ID NO: 168 | 18 | 21 |
| p21M-155 | Mini D21S168-F | * | TGTAGGCTGTTTAGTTGGTGGA | SEQ ID NO:169 | 22 | 21 |
| p21M-156 | Mini D21S168-R | | * CGGCATCACAGTCTGATAAAA | SEQ ID NO: 170 | 21 | 21 |
| p21M-157 | Mini D21S210-F | * | TGATAAGCCTCCCTCACTACTATTTT | SEQ ID NO: 171 | 26 | 21 |
| p21 M-158 | Mini D21S210-R | | GCAGCGATAGCTAGTCATAGTGAA | SEQ ID NO: 172 | 24 | 21 |
| p21M-159 | Mini D21S214-F | * | CCTGCAAGGACACCAAGTTA | SEQ ID NO: 173 | 20 | 21 |
| p21 M-160 | Mini D21S214-R | | TGTTCACCTGATTTTCGGTTC | SEQ ID NO: 174 | 21 | 21 |
| p21M-161 | Mini GATA116E08-F | * | TGCAAGCCACATCATTTGT | SEQ ID NO: 175 | 19 | 21 |
| p21M-162 | Mini GATA116E08-R | | TCGAATCGATAGATAGATAGGTGA | SEQ ID NO: 176 | 24 | 21 |
| p21M-163 | Mini GATA148F04-F | * | TGAAACAAGGGAATCTATCATC | SEQ ID NO: 177 | 22 | 21 |
| p21M-164 | Mini GATA148F04-R | | TGATAAATAGTGAATATAGTTGACAGC | SEQ ID NO: 178 | 27 | 21 |
| p21M-165 | Mini GATA163G03-F | * | TTGTGGGGCCTTGTAATTGT | SEQ ID NO: 179 | 20 | 21 |
| p21 M-166 | Mini GATA163G03-R | | CAGGGTCCCTAGAGAGACAGA | SEQ ID NO: 180 | 21 | 21 |
| p21M-167 | Mini D21S2055-F | * | CAGAACCAATAGGCTATCTATCT | SEQ ID NO: 181 | 23 | 21 |
| p21M-168 | Mini D21S2055-R | | ACAGTAAATCACTTGGTAGGAGA | SEQ ID NO: 182 | 23 | 21 |
| p21M-169 | Mini D21S1442-F | * | GGGCACCCCTTTATACTTGG | SEQ ID NO: 183 | 20 | 21 |
| p21M-170 | Mini D21S1442-R | | TCACATGAGCCAATTCCTTATAATAG | SEQ ID NO: 184 | 26 | 21 |
| p21M-171 | Mini GATA29C02-F | * | TCTATACATATGTGTGTGTGCAT | SEQ ID NO: 185 | 23 | 21 |
| p21M-172 | Mini GATA29C02-R | | CACCTTTGTTGCCAAGAGTC | SEQ ID NO: 186 | 20 | 21 |
| p21M-173 | Mini GATA29D01-F | * | TTCTGTTAAATGAGTAAGGAGATGACA | SEQ ID NO: 187 | 27 | 21 |
| p21M-174 | Mini GATA29D01-R | | GCATGCGTGTGTGTGTGTAT | SEQ ID NO: 188 | 20 | 21 |
| p21M-175 | Mini D21S1433-F | * | GAGCTGAGATCACGACAGTCA | SEQ ID NO: 189 | 21 | 21 |
| p21M-176 | Mini D21S1433-R | | TATTTTCAGGCCAAGCCTTT | SEQ ID NO: 190 | 20 | 21 |
| p21M-177 | Mini GATA45C03-F | * | GAAACAGAACTAATAGGATCTATCTGC | SEQ ID NO: 191 | 27 | 21 |
| p21M-178 | Mini GATA45C03-R | | GGCAAACAAATAGTTGATAGATGAG | SEQ ID NO: 192 | 25 | 21 |
| p21M-179 | Mini D21S1446-F | * | TGACCATCTTACTGGTTTATGTATTT | SEQ ID NO: 193 | 26 | 21 |
| p21M-180 | Mini D21S1446-R | | CGAGGCTATTTTACTGGTAACTAACTG | SEQ ID NO: 194 | 27 | 21 |
| p21M-181 | Mini D21S1270-F | * | GGGCTACATAGAGAAACAGAACC | SEQ ID NO: 195 | 23 | 21 |
| p21M-182 | Mini D2151270-R | | ACACACACACACACACATGC | SEQ ID NO: 196 | 20 | 21 |
| p21M-183 | Mini D21S1436-F | * | GGAAAGAGAAAGAAAGGAAGGAA | SEQ ID NO: 197 | 23 | 21 |
| p21M-184 | Mini D21S1436-R | | CCATTTATGTCCTATTTCCTACTCC | SEQ ID NO: 198 | 25 | 21 |
| p21M-185 | Mini D21S1265-F | * | GGACTCCTCCAGCTGAACTCT | SEQ ID NO: 199 | 21 | 21 |
| p21M-186 | Mini D21S1265-R | | GCACAGTACAGCAAACTTGTCA | SEQ ID NO: 200 | 22 | 21 |
| p21M-187 | Mini D21S1249-F | * | TTTCCACACGGCTAATCTACTT | SEQ ID NO: 201 | 22 | 21 |
| p21M-1888 | Mini D21S1249-R | | TACCTCCCTCCCTCCATCC | SEQ ID NO: 202 | 19 | 21 |
| p21M-189 | Mini D21S1409-F | * | GGGGAATACATTTGTGTAGGTAGG | SEQ ID NO: 203 | 24 | 21 |
| p21M-190 | Mini D21S1409-R | | CACTAATACCTGGTGAATGATTCTT | SEQ ID NO: 204 | 25 | 21 |
| p21M-191 | Mini D21S1410-F | * | AAATGAAGATATTTTCTTAGCTTAT | SEQ ID NO: 205 | 25 | 21 |
| p21M-192 | Mini D2151410-R | | GCATTCAATATTTTACTTTTAAGAATC | SEQ ID NO: 206 | 27 | 21 |
| p21M-193 | Mini D21S1250-F | * | GGGTAAAGAAAATGTGCTCTCTC | SEQ ID NO: 207 | 23 | 21 |
| p21M-194 | Mini D21S1250-R | | GGTTCTCCAAGTTCAATGGTG | SEQ ID NO: 208 | 21 | 21 |
| p21M-195 | Mini D21S1251-F | * | CAGCAGAAAGGGAATAGTTGG | SEQ ID NO: 209 | 21 | 21 |
| p21M-196 | Mini D21S1251-R | | CAAGTTAAAAACACAAAATGGAAA | SEQ ID NO: 210 | 24 | 21 |
| p21M-197 | Mini D21S1411-F | * | TGGATGGATGGATAGATACACAG | SEQ ID NO:211 | 23 | 21 |
| p21M-198 | Mini D21S1411-R | | CCCACTCCCAGCCTTCTAA | SEQ ID NO: 212 | 19 | 21 |
| p21M-199 | Mini D21S1238-F | * | TCTCTGTGTCTATGTGTGCATGTT | SEQ ID NO: 213 | 24 | 21 |
| p21M-200 | Mini D21S1238-R | | GGTTTTGCAAAGGCAGGTTA | SEQ ID NO: 214 | 20 | 21 |
| p21M-201 | Mini D2151239-F | * | CACTTTCACAATATGTATTGCTTATCA | SEQ ID NO: 215 | 27 | 21 |
| p21M-202 | Mini D21S1239-R | | GGTAGCAATTTCACTCTCTCTTTC | SEQ ID NO: 216 | 24 | 21 |
| p21M-203 | Mini D21S1408-F | * | GATGACAAGACAGATTAGATAGATTGG | SEQ ID NO: 217 | 27 | 21 |
| p21M-204 | Mini D21S1408-R | | CATTGGGCTTATTTTTCTTTCTAT | SEQ ID NO: 218 | 24 | 21 |
| p21M-205 | Mini UT556-F | * | AAAGGCAGGAAGGCAGGA | SEQ ID NO: 219 | 18 | 21 |
| p21M-206 | Mini UT556-R | | TTTTCTTCTTTTTGCTTCTCTTTTC | SEQ ID NO: 220 | 25 | 21 |
| p21M-207 | Mini D21S1240-F | * | TGATGTAGTTCACTAGGATGTAGGG | SEQ ID NO:221 | 25 | 21 |
| p21M-208 | Mini D21S1240-R | | CCTGAGACACAAGAGCGAGA | SEQ ID NO:222 | 20 | 21 |
| p21M-209 | Mini D21S1412-F | * | CCTGGGTGACAAGAGTGAAA | SEQ ID NO: 223 | 20 | 21 |
| p21M-210 | Mini D21S1412-R | | CACAGAAATTTGTAGAACCACAGC | SEQ ID NO: 224 | 24 | 21 |
| p21M-211 | Mini D21S1280-F | * | GGCATCAAAAMGGAAGAAA | SEQ ID NO: 225 | 21 | 21 |
| p21M-212 | Mini D21S1280-R | | AAAGCTGAGCTGAATGGTGA | SEQ ID NO: 226 | 20 | 21 |
| p21M-213 | Mini D21S1413-F | * | GGGAAACCACAGTTATACATTCC | SEQ ID NO: 227 | 23 | 21 |
| p21M-214 | Mini D2151413-R | | TGTTTACAGTTCTTCACAGAGTTCTT | SEQ ID NO: 228 | 26 | 21 |
| p21M-215 | Mini D21S1244-F | * | ACCACAGAATTCAGTCCAAAAA | SEQ ID NO: 229 | 22 | 21 |
| p21M-216 | Mini D21S1244-R | | TTATCCCCTGGAGGAACTTG | SEQ ID NO: 230 | 20 | 21 |
| p21M-217 | Mini D21S1245-F | * | CCAGAAAATGACACATGAAGGA | SEQ ID NO: 231 | 22 | 21 |
| p21M-218 | Mini D21S1245-R | | GAGATATTGGCCTGTAGTTTTCTTTT | SEQ ID NO: 232 | 26 | 21 |
| p21M-219 | Mini D21S1414-F | * | GATGTTGTATTAGTCAATGTTCTCCA | SEQ ID NO: 233 | 26 | 21 |
| p21M-220 | Mini D21S1414-R | | TCTGTCTGTCTGTCTGTCTGTCTG | SEQ ID NO: 234 | 24 | 21 |
| p21M-221 | Mini D21S1246-F | * | ATGGGCAAACAGATGGGTAG | SEQ ID NO: 235 | 20 | 21 |
| p21M-222 | Mini D21S1246-R | | CCATATTATCATCCATCCATCCA | SEQ ID NO: 236 | 23 | 21 |

**Table 3: Mini-STR Primers for Chromosome 13**

| *Note: * 6-FAM. VIC. NED. JOE. RON. PET. TAMBA. or 5-FAM.* | | | | | | |
|---|---|---|---|---|---|---|
| **Number** | **Name/Loci** | **5' Label** | **Sequence** | **Seq. ID No.** | **Length** | **Chromosome** |
| p13M-1 | Mini D13S1236-F | * | GGGTAACAGCATAAGACCCTGT | SEQ ID NO: 237 | 22 | 13 |
| p13M-2 | Mini D13S1236-R | | TCACTTTGGTGCTTGCTTTG | SEQ ID NO: 238 | 20 | 13 |
| p13M-3 | Mini D13S175-F | * | GAATCTGCTGAGAGAGTAGATTTTAAG | SEQ ID NO: 239 | 27 | 13 |
| p13M-4 | Mini D13S175-R | | TGCATCACCTCACATAGGTTACT | SEQ ID NO: 240 | 23 | 13 |
| p13M-5 | Mini D13S1243-F | * | TGCTGACAGGCTACAGAACTTT | SEQ ID NO: 241 | 22 | 13 |
| p13M-6 | Mini D1351243-R | | TCTGCATTRGTAGAAATAATCTTATCA | SEQ ID NO: 242 | 27 | 13 |
| p13M-7 | Mini D13S1304-F | * | ACCATTATTCTCCTGAGTCCTCTC | SEQ ID NO: 243 | 24 | 13 |
| p13M-8 | Mini D13S1304-R | | ACATTCTAGTGCTACAGGGTATTC | SEQ ID NO: 244 | 24 | 13 |
| p13M-9 | Mini D13S289-F | * | GTCCCACTATCTCAATAATCTGATG | SEQ ID NO: 245 | 25 | 13 |
| p13M-10 | Mini D13S289-R | | ACTGGTCACCTTCATCACCA | SEQ ID NO: 246 | 20 | 13 |
| p13M-11 | Mini D13S171-F | * | GGAGAAAGGGGAGGTGTAGA | SEQ ID NO: 247 | 20 | 13 |
| p13M-12 | Mini D13S171-R | | CCATCCTCCTCCCTTCTTTTT | SEQ ID NO: 248 | 21 | 13 |
| p13M-13 | Mini D13S219-F | * | TTGCCATGTCAATTGCTACA | SEQ ID NO: 249 | 20 | 13 |
| p13M-14 | Mini D13S219-R | | TGTTTCTTGACTTAACATTTTCTTCT | SEQ ID NO: 250 | 26 | 13 |
| p13M-15 | Mini D13S218-F | * | GATTTGAAAATGAGCAGTCCA | SEQ ID NO: 251 | 21 | 13 |
| p13M-16 | Mini D13S218-R | | GCATGTTTCAGGTCTTTTATTGC | SEO ID NO: 252 | 23 | 13 |
| p13M-17 | Mini D13S263-F | * | GCCTGTTAGTTTTTATTGTTATCTTAG | SEQ ID NO: 253 | 27 | 13 |
| p13M-18 | Mini D13S263-R | | TTTTTATCAGAAGCATGAAAACAG | SEQ ID NO: 254 | 24 | 13 |
| p13M-19 | Mini D13S153-F | * | CTGTTTCTCCTCCCTGCAAC | SEQ ID NO: 255 | 20 | 13 |
| p13M-20 | Mini D13S153-R | | GGAGCGTATCTGTGCGTGTA | SEQ ID NO: 256 | 20 | 13 |
| p13M-21 | Mini D13S1320-F | * | CACTTCTAGGTTTTTACCCAAGTGA | SEQ ID NO:257 | 25 | 13 |
| p13M-22 | Mini D13S1320-R | | TGAAGTAACTCTGAACACTCAATACTT | SEQ ID NO: 258 | 27 | 13 |
| p13M-23 | Mini D13S1296-F | * | GTTTAACCAGGAGCCCTTCC | SEQ ID NO: 259 | 20 | 13 |
| p13M-24 | Mini D13S1296-R | | GAGCAACTACCTACTATGGTTCCTT | SEQ ID NO: 260 | 25 | 13 |
| p13M-25 | Mini D13S156-F | * | ACTCCAGCCTGGGCGATAG | SEQ ID NO: 261 | 19 | 13 |
| p13M-26 | Mini D13S156-R | | CTTGGATTTATGTATCTCTCCTAGAGT | SEQ ID NO: 262 | 27 | 13 |
| p13M-27 | Mini D13S1306-F | * | GCTGTTCTTCTAAGTGCCACA | SEQ ID NO: 263 | 21 | 13 |
| p13M-28 | Mini D13S1306-R | | GGGGTTGTTGCGAAGATTAG | SEQ ID NO: 264 | 20 | 13 |
| p13M-29 | Mini D13S170-F | * | TGGAGATAAACACATAGGCACA | SEQ ID NO: 265 | 22 | 13 |
| p13M-30 | Mini D13S170-R | | TAAGGCAGGAGTCATGTCCA | SEQ ID NO: 266 | 20 | 13 |
| p13M-31 | Mini D13S265-F | * | GCCAATTACATTGCATATTGCAT | SEQ ID NO: 267 | 23 | 13 |
| p13M-32 | Mini D13S265-R | | CAACAAAGCAATAAAGAGTTTTGC | SEQ ID NO 268 | 24 | 13 |
| p13M-33 | Mini D13S1241-F | * | ATGGAGTGCCACTGGAAGAA | SEQ ID NO: 269 | 20 | 13 |
| p13M-34 | Mini D13S1241-R | | CCAGTTGAGTTTGGACCTCAG | SEQ ID NO: 270 | 21 | 13 |
| p13M-35 | Mini D13S159-F | * | GGCCAAAATTAGCGTGACA | SEQ ID NO: 271 | 19 | 13 |
| p13M-36 | Mini D13S159-R | | CAACTCCAGGCCAAATCATC | SEQ ID NO: 272 | 20 | 13 |
| p13M-37 | Mini D13S158-F | * | CGGAGTGAAAGAAGATTGATTT | SEQ ID NO: 273 | 22 | 13 |
| p13M-38 | Mini D13S158-R | | TTGACAATTTAGCAGCATGTATTT | SEQ ID NO: 274 | 24 | 13 |
| p13M-39 | Mini D13S173-F | * | AGCCTCATGCCTGGGGATA | SEQ ID NO: 275 | 19 | 13 |
| p13M-40 | Mini D13S173-R | | ATTTTCTTCATTTGGTGTTATTTTGG | SEQ ID NO: 276 | 26 | 13 |
| p13M-41 | Mini D13S1265-F | * | CTTTTCAGATRAAATGAGACAATATG | SEQ ID NO: 277 | 26 | 13 |
| p13M-42 | Mini D13S1265-R | | TGCTAATGTGTGATTATATGTACGC | SEQ ID NO: 278 | 25 | 13 |

**Table 4: Mini-STR Primers for Chromosome 18**

| *Note: * 6-FAM. VIC. NED. JOE. RON. PET. TAMBA. or 5-FAM.* | | | | | | |
|---|---|---|---|---|---|---|
| **Number** | **Name/Loci** | **5' Label** | **Sequence** | **Seq. ID No.** | **Length** | **Chromosome** |
| p18M-1 | Mini D18S51-F | * | TGAGTGACAAATTGAGACCTT | SEQ ID NO: 279 | 21 | 18 |
| p18M-2 | Mini D18S51-R | | GTCTTACAATAACAGTTGCTACTATT | SEQ ID NO: 280 | 26 | 18 |
| p18M-3 | Mini D18S59-F | * | AACAGGGGCACAAGACAGAT | SEQ ID NO: 281 | 20 | 18 |
| p18M-4 | Mini D18S59-R | | CCCACTCCTGTGCACTCTCT | SEQ ID NO: 282 | 20 | 18 |
| p18M-5 | Mini D18S476-F | * | GTTGACAATAGCACACATACAGTCC | SEQ ID NO: 283 | 25 | 18 |
| p18M-6 | Mini D18S476-R | | ACCACCACCCACACCATC | SEQ ID NO:284 | 18 | 18 |
| p18M-7 | Mini D18S63-F | * | TCTTCCATTCCCATCATTTCA | SEQ ID NO: 285 | 21 | 18 |
| p18M-8 | Mini D18S63-R | | TCTCCAGGAACATTGTTTTACTTT | SEQ ID NO: 286 | 24 | 18 |
| p18M-9 | Mini D18S1132-F | * | CAGCCTTTTCCAAATTTTACATC | SEQ ID NO: 287 | 23 | 18 |
| p18M-10 | Mini D18S1132-R | | TGCCCTACCAGACCAATTTT | SEQ ID NO: 288 | 20 | 18 |
| p18M-11 | Mini D18S452-F | * | TGGGGCATACATAGTGCAAA | SEQ ID NO: 289 | 20 | 18 |
| p18M-12 | Mini D18S452-R | | AAATAACCGCTGGCTCTGTG | SEQ ID NO: 290 | 20 | 18 |
| p18M-13 | Mini D18S464-F | * | GACTTTGTGCCATTTCTCCA | SEQ ID NO: 291 | 20 | 18 |
| p18M-14 | Mini D18S464-R | | AATCTTCAGGCTGCTTGCAC | SEQ ID NO: 292 | 20 | 18 |
| p18M-15 | Mini D18S1150-F | * | GGCACAGGAAACGTGAATTT | SEQ ID NO: 293 | 20 | 18 |
| p18M-16 | Mini D18S1150-R | | GCTGTTTTCTTCTGTTGTCTGG | SEQ ID NO: 294 | 22 | 18 |
| p18M-17 | Mini D18553-F | * | TTTGGATGTCTTTCTTTCTTCTATCA | SEQ ID NO: 295 | 26 | 18 |
| p18M-18 | Mini D18S53-R | | CAGTGGAAACCAAACTACAACG | SEQ ID NO: 296 | 22 | 18 |
| p18M-19 | Mini D18S453-F | * | CAATAAAGACCTGACTTGGAAAAA | SEQ ID NO: 297 | 24 | 18 |
| P18M-20 | Mini D18S453-R | | CTCAACACAGCAACAAAAATATAAA | SEQ ID NO: 298 | 25 | 18 |
| p18M-21 | Mini D18S478-F | * | AAGAGAAGAACATCACTAAGAACCA | SEQ ID NO: 299 | 25 | 18 |
| p18M-22 | Mini D18S478-R | | AACTCAGTGTTCCACAGTAACTCA | SEQ ID NO: 300 | 24 | 18 |
| p18M-23 | Mini D18S56-F | * | CTGAAGGACCTGCCTGAGAT | SEQ ID NO: 301 | 20 | 18 |
| p18M-24 | Mini D18S56-R | | TACTTTTTATTGTTAGGGTGTGCTC | SEQ ID NO: 302 | 25 | 18 |
| p18M-25 | Mini D18S468-F | * | CCCCTGCATAAACTCACTCA | SEQ ID NO: 303 | 20 | 18 |
| p18M-26 | Mini D18S468-R | | TTCCAAAGGACATAATCCATATTT | SEQ ID NO: 304 | 24 | 18 |
| p18M-27 | Mini D18S450-F | * | GGACCTAGGTTCCAATTTCTCC | SEQ ID NO: 305 | 22 | 18 |
| P18M-28 | Mini D18S450-R | | TGTATGGTGCATGAACCTGTG | SEQ ID NO: 306 | 21 | 18 |
| p18M-29 | Mini D18S474-F | * | CTGGCCTCCACCCACTAGAT | SEQ ID NO: 307 | 20 | 18 |
| p18M-30 | Mini D18S474-R | | CTTTCAATGTCAGAAGGCATTT | SEQ ID NO: 308 | 22 | 18 |
| P18M-31 | Mini D18S1127-F | * | ACCCTGGAGAGTGACTGCAT | SEQ ID NO: 309 | 20 | 18 |
| p18M-32 | Mini D18S1127-R | | CGCCTGTACTGCCTGAGTTT | SEQ ID NO: 310 | 20 | 18 |
| p18M-33 | Min D18S1129-F | * | GGCTGCACAGGCATTC | SEQ ID NO: 311 | 16 | 18 |
| p18M-34 | Mini D18S1129-R | | GGGAATGCAGTGAAATGGAC | SEQ ID NO: 312 | 20 | 18 |
| p18M-35 | Mini D18S64-F | | TTTTGCCACAAAAATTACCAA | SEQ ID NO: 313 | 21 | 18 |
| p18M-36 | Mini D18564-R | * | AAATCAGGAAATCGGCACTG | SEQ ID NO: 314 | 20 | 18 |
| p18M-37 | Min D18S1147-F | * | TCAGCACAATGCTACTGGGTA | SEQ ID NO: 315 | 21 | 18 |
| p18M-38 | Mini D18S1147-R | | GACTGGGAACATGGCTCTTC | SEQ ID NO: 316 | 20 | 18 |
| p18M-39 | Mini D18S68-F | * | TGTGGAAAGTTGTAGATAGGATGAA | SEQ ID NO: 317 | 25 | 18 |
| p18M-40 | Mini D18S68-R | | TGAGGATCACACTTTGAGTAGTAAGTC | SEQ ID NO: 318 | 27 | 18 |
| p18M-41 | Mini D18S61-F | * | CCAAACTACATTCTTCTTCCTGA | SEQ ID NO: 319 | 23 | 18 |
| p18M-42 | Mini D18S61-R | | GAGGAATTTATGCTAAGATTTGAAGG | SEQ ID NO: 320 | 26 | 18 |
| p18M-43 | Mini D185469-F | * | AACACGCTTGTCAAATGCTT | SEQ ID NO: 321 | 20 | 18 |
| p18M-44 | Mini D18S469-R | | TTAAGTTATTGTTGTTGTTTCTTGTGG | SEQ ID NO: 322 : | 27 | 18 |
| p18M-45 | Mini D18S462-F | * | CAGAAGCAGATTTGAACATTGG | SEQ ID NO: 323 | 22 | 18 |
| p18M-46 | Mini D18S462-R | | GCTATAAACATTCACCGTTAGGG | SEQ ID NO: 324 | 23 | 18 |
| p18M-47 | Mini D18S70-F | * | GGCCTCTCTCCCAGAAAGAT | SEQ ID NO: 325 | 20 | 18 |
| p18M-48 | Mini D18S70-R | | TGTCAAGAAGTACCTACCATATTTTGA | SEQ ID NO: 326 | 27 | 18 |

**Table 5: Mini-STR Primers for Chromosome X**

| *Note: * - 6-FAM, NED, JOE, ROX, PET, TAMRA or 5-FAM* | | | | | | |
|---|---|---|---|---|---|---|
| **Number** | **Name/Loci** | **5' Label** | **Sequence** | **Seq. ID No.** | **Length** | **Chromosome** |
| pXM-1 | Mini DXS1060-F | * | CTCCCTCTTAATGTTGCCTGT | SEQ ID NO: 327 | 21 | X |
| pXM-2 | Mini DXS1060-R | | TGAGAGTCTTTGGTGGGAGA | SEQ ID NO: 328 | 20 | X |
| pXM-3 | Mini DXS1223-F | * | TGCTTTTGGTGTCTTCAATCTG | SEQ ID NO: 329 | 22 | X |
| pXM-4 | Mini DXS1223-R | | TGGTCATGTAACAGTGCTTGG | SEQ ID NO: 330 | 21 | X |
| pXM-5 | Mini DXS8051-F | * | TGACATTTAATCAACCAAGAAAT | SEQ ID NO: 331 | 23 | X |
| pXM-6 | Mini DXS8051-R | | TTTTTGAACTAAGAACCTGGAG | SEQ ID NO: 332 | 22 | X |
| pXM-7 | Mini DXS7108-F | * | TTGTTAGTGTTTGCAAAGTGATGA | SEQ ID NO: 333 | 24 | X |
| pXM-8 | Mini DXS7108-R | | GGATTTATAGATATGGGAGGGTTC | SEQ ID NO: 334 | 24 | X |
| pXM-9 | Mini DXS1224-F | * | CCCTTGATGTAGGCACAGG | SEQ ID NO: 335 | 19 | X |
| pXM-10 | Mini DXS1224-R | | CGTGGGGGAGTAGTAGTGGT | SEQ ID NO: 336 | 20 | X |
| pXM-11 | Mini DXS8019-F | * | CTTCTTGCATTCCCCATGC | SEQ ID NO: 337 | 19 | X |
| pXM-12 | Mini DXS8019-R | | TTTCCTCACAGCAAAAGAGG | SEQ ID NO: 338 | 20 | X |
| pXM-13 | Mini DXS7593-F | * | CCTGGGCAACAAGAGTGAA | SEQ ID NO: 339 | 19 | X |
| pXM-14 | Mini DXS7593-R | | GGAAAGAGAGTTATATTTAAGAGCAGA | SEQ ID NO: 340 | 27 | X |
| pXM-15 | Mini DXS1226-F | * | CCCATCTGTCCTCCTGGATA | SEQ ID NO: 341 | 20 | X |
| pXM-16 | Mini DXS1226-R | | GGTCCCCTATTTGTCTTGTCC | SEQ ID NO: 342 | 21 | X |
| pXM-17 | Mini DXS1061-F | * | TCCCTTTCTCTCTCTCTCTCTCTC | SEQ ID NO: 343 | 24 | X |
| pXM-18 | Mini DXS1061-R | | TGATGTGTTATGAATTGGCAAAA | SEQ ID NO: 344 | 23 | X |
| pXM-19 | Mini DXS1214-F | * | GGTTGGAATGACTGAAGGCTTA | SEQ ID NO: 345 | 22 | X |
| pXM-20 | Mini DXS1214-R | | AAGATAGCAGGCAACAATAAGAT | SEQ ID NO: 346 | 23 | X |
| pXM-21 | Mini DXS1068-F | * | GTTCTAGGGACACTCCCTTC | SEQ ID NO: 347 | 21 | X |
| pXM-22 | Mini DXS1068-R | | AGACCATGGCCTGCTTTTA | SEQ ID NO: 348 | 19 | X |
| pXM-23 | Mini DXS8015-F | * | GCCTTACACACAAGCACACC | SEQ ID NO: 349 | 20 | X |
| pXM-24 | Mini DXS8015-R | | GCACCAATATCAAAGCAGCA | SEQ ID NO: 350 | 20 | X |
| pXM-25 | Mini DXS993-F | * | ACCACTCAGCCAGTTTGCTT | SEQ ID NO: 351 | 20 | X |
| pXM-26 | Mini DXS993-R | | GAACTGGCCTTGCCTTCAC | SEQ ID NO: 352 | 19 | X |
| pXM-27 | Mini DXS8080-F | * | GGGCAACAAGAGCAAAACTC | SEQ ID NO: 353 | 20 | X |
| pXM-28 | Mini DXS8080-R | | CCCTGTTGGTAAATCCTTGG | SEQ ID NO: 354 | 20 | X |
| pXM-29 | Mini DXS8083-F | * | CAAGGAACTCAAACAACAGTTTACA | SEQ ID NO: 355 | 25 | X |
| pXM-30 | Mini DXS8083-R | | TCTTTGCCCACTTTTTAATGG | SEQ ID NO: 356 | 21 | X |
| pXM-31 | Mini DXS991-F | * | GGTTCTCCAGAGGGACAGAA | SEQ ID NO: 357 | 20 | X |
| pXM-32 | Mini DXS991-R | | TCTCCCTGATAAACTCCTTTTCAT | SEQ ID NO: 358 | 24 | X |
| pXM-33 | Mini DXS1216-F | * | TCTCTTTCAGTGACCCCTCCT | SEQ ID NO: 359 | 21 | X |
| pXM-34 | Mini DXS1216-R | | GGGGAAAGAGAGAGAGAGGAA | SEQ ID NO: 360 | 21 | X |
| pXM-35 | Mini DXS986-F | * | CCACAAGCAGATAAAGAAAATGTG | SEQ ID NO: 361 | 24 | X |
| pXM-36 | Mini DXS986-R | | TCATTTTTATGGCCATGGTATGT | SEQ ID NO: 362 | 23 | X |
| pXM-37 | Mini DXS1196-F | * | TATTTCCCCCAGCACCCTTT | SEQ ID NO: 363 | 20 | X |
| pXM-38 | Mini DXS1196-R | | TTTCAGTAAAATCATACACCTTTAACA | SEQ ID NO: 364 | 27 | X |
| pXM-39 | Mini DXS1217-F | * | ATCTTTGGAGGGGAAGGAGT | SEQ ID NO: 365 | 20 | X |
| pXM-40 | Mini DXS1217-R | | GAAGTATCGTATCTGAATCCCGTA | SEQ ID NO: 366 | 24 | X |
| pXM-41 | Mini DXS8020-F | * | TTCAAAGAGCCCTCTGCTGT | SEQ ID NO: 367 | 20 | X |
| pXM-42 | Mini DXS8020-R | | ACAATTCTGTATAGACTTTGTGTGT | SEQ ID NO: 368 | 25 | X |
| pXM-43 | Mini DXS1106-F | * | TGAGAACTCCCTAAACAAAATGT | SEQ ID NO: 369 | 23 | X |
| pXM-44 | Mini DXS1106-R | | TTCCTTGAATGTAAGGATTAGGG | SEQ ID NO: 370 | 23 | X |
| pXM-45 | Mini DXS1059-F | * | TTTGCCTACCACGGTTGTCT | SEQ ID NO: 371 | 20 | X |
| pXM-46 | Mini DXS1059-R | | ACCCGTCGTGGTTGTGAT | SEQ ID NO: 372 | 18 | X |
| pXM-47 | Mini DXS8088-F | * | TCCTGTTTTCCAGTACCAGAAGT | SEQ ID NO: 373 | 23 | X |
| pXM-48 | Mini DXS8088-R | | GAGTCTATTAGGAGCACAAAAAGG | SEQ ID NO: 374 | 24 | X |
| pXM-49 | Mini DXS8055-F | * | TTGACTAGAAATGCTCCCTCAA | SEQ ID NO: 375 | 22 | X |
| pXM-50 | Mini DXS8055-R | | CAGGTTTCTGTGTGGACATTG | SEQ ID NO: 376 | 21 | X |
| pXM-51 | Mini DXS8064-F | * | ACTCCAGCCTGAGCAACAG | SEQ ID NO: 377 | 19 | X |
| pXM-52 | Mini DXS8064-R | | CATTGCTCCCCCAACAACT | SEQ ID NO: 378 | 19 | X |
| pXM-53 | Mini DXS8067-F | * | GAGGGCAACAGAGTGGAGAC | SEQ ID NO: 379 | 20 | X |
| pXM-54 | Mini DXS8067-R | | TGATTTTGTACACATTTATGGGGTAT | SEQ ID NO: 380 | 26 | X |
| pXM-55 | Mini DXS1001-F | * | CCTTCACATGTATCCCCAAA | SEQ ID NO: 381 | 20 | X |
| pXM-56 | Mini DXS1001-R | | TGAATGGATAAAGAAAATGTGGT | SEQ ID NO: 382 | 23 | X |
| pXM-57 | Mini DXS8009-F | * | AAACTGTGGAAATTGCTTCCAT | SEQ ID NO: 383 | 22 | X |
| pXM-58 | Mini DXS8009-R | | TCAACAAATTCCAGGTTATGTCA | SEQ ID NO: 384 | 23 | X |
| pXM-59 | Mini DXS1047-F | * | TTTTAAAAACTTCTACAATGAGCA | SEQ ID NO: 385 | 24 | X |
| pXM-60 | Mini DXS1047-R | | CCTAGGTAACATAGTGAGACCTTGTC | SEQ ID NO: 386 | 26 | X |
| pXM-61 | Mini DXS1062-F | * | ATGATGCCTGGCACACAGTA | SEQ ID NO: 387 | 20 | X |
| pXM-62 | Mini DXS1062-R | | AAGCACTTTGAATCATTTACGG | SEQ ID NO: 388 | 22 | X |
| pXM-63 | Mini DXS984-F | * | ACCCCCACCTCCCTGAAATA | SEQ ID NO: 389 | 20 | X |
| pXM-64 | Mini DXS984-R | | TGCCCTACTCCATTCCACAC | SEQ ID NO: 390 | 20 | X |
| pXM-65 | Mini DXS1205-F | * | CCACTTGTCCTCTTGCTACACA | SEQ ID NO: 391 | 22 | X |
| pXM-66 | Mini DXS1205-R | | TGGCTTAGAGTACTTTTTCACTGC | SEQ ID NO: 392 | 24 | X |
| pXM-67 | Mini DXS1227-F | * | TCCAAAATAACACTGAAACACG | SEQ ID NO: 393 | 22 | X |
| pXM-68 | Mini DXS1227-R | | AAGGGTTTACTCCCCCAAAA | SEQ ID NO: 394 | 20 | X |
| pXM-69 | Mini DXS8106-F | * | GGTATAAAACTGAACTCATCAGCA | SEQ ID NO: 395 | 24 | X |
| pXM-70 | Mini DXS8106-R | | AGCTGTAGAGTTGAGGAATGTTTTC | SEQ ID NO: 396 | 25 | X |
| pXM-71 | Mini DXS8043-F | * | AAACATTTGGTTAGGCTAATTTCTAT | SEQ ID NO: 397 | 26 | X |
| pXM-72 | Mini DXS8043-R | | AAACAAATGCGAATTTAAAAAGA | SEQ ID NO: 398 | 23 | X |
| pXM-73 | Mini DXS8045-F | * | GGAGATTTCTTCCTTGTTGCAC | SEQ ID NO: 399 | 22 | X |
| pXM-74 | Mini DXS8045-R | | GCTAGGCTGTGTGTGTCTGTG | SEQ ID NO: 400 | 21 | X |
| pXM-75 | Mini DXS998-F | * | AAAGGCAAAGAAAAACTGTTGC | SEQ ID NO: 401 | 22 | X |
| pXM-76 | Mini DXS998-R | | GATCATTCATATAACCTCAAAAGAACT | SEQ ID NO: 402 | 27 | X |
| pXM-77 | Mini DXS8069-F | * | GGCATCGTATTCATTGTTCCA | SEQ ID NO: 403 | 21 | X |
| pXM-78 | Mini DXS8069-R | | AGGTTCTTCCAAATTATTTTTGTG | SEQ ID NO: 404 | 24 | X |
| pXM-79 | Mini DXS1073-F | * | TGAAACACTGCTCCCCTTG | SEQ ID NO: 405 | 19 | X |
| pXM-80 | Mini DXS1073-R | | CCGAGTTATTACAAAGAAGCACA | SEQ ID NO: 406 | 23 | X |

## Claims

1. A method for conducting a genetic test of a fetus comprising
isolating, by size fractionation, a nucleic acid sample from a cervical mucus sample obtained from a female subject containing the fetus, the nucleic acid sample consisting essentially of polynucleotides in a size ranging from about 50 base pairs to about 300 base pairs, and
wherein the result of a genetic test on the nucleic acid sample is indicative of a genetic composition of the fetus.

2. The method of claim 1, wherein the cervical mucus sample is obtained by transcervical swabs, endocervical lavage, cytobrush, aspiration, intrauterine lavage, or a combination thereof.

3. The method of claim 1, wherein the nucleic acid sample is a DNA sample or an RNA sample.

4. The method of claim 1, further comprising using the isolated nucleic acid sample to test for a genetic composition not uniquely associated with Y chromosome, wherein the genetic composition of the isolated nucleic acid sample is indicative of the genetic composition of the fetus.

5. The method of claim 4, wherein the genetic composition is selected from the group consisting of trisomy, partial trisomy, chromosomal duplication, monosomy, partial monosomy, chromosomal deletion, chromosomal translocation, and chromosomal inversion.

6. The method of claim 4, wherein the genetic composition is indicative of a disease condition selected from the group consisting of Down Syndrome, Edwards Syndrome, Patau Syndrome, Fragile X Syndrome, Turner Syndrome, Klinefelter's Syndrome, Triple X syndrome, XYY syndrome, Trisomy 8, Trisomy 16, Wolf-Hirschhorn Syndrome, and RhD Syndrome.

7. The method of claim 1, further comprising using the isolated nucleic acid sample as a template for a genetic test using an assay technology selected from the group consisting of PCR, real-time PCR, LCR, Q-B-replicase, SDA, RCA, TMA, LADA, MDA, and invader.

8. The method of claim 1, further comprising using the isolated nucleic acid sample to test the presence of a genetic marker or an allele in the fetus, wherein the presence of the genetic marker or allele is based on the amplification of a nucleotide fragment using a pair of primers specific for the genetic marker or allele.

9. The method of claim 8, wherein the allele corresponds to a genetic condition selected from the group consisting of sickle-cell anemia, Phenylketonuria, Tay-Sachs disease, Cystic Fibrosis, beta-Thalassemia, Adrenal Hyperplasia, Fanconi Anemia, Spinal Muscularatrophy, Duchenne's Muscular Dystrophy, Huntington's Disease, Myotonic Dystrophy, Robertsonian translocation, Angelman syndrome, DiGeorge Syndrome, Tuberous Sclerosis, Ataxia Telangieltasia, and Prader-Willi syndrome.

10. The method of claim 8, wherein the pair of primers is selected from the group consisting of primers of SEQ ID NOs: 17 and 18, SEQ ID NOs: 15 and 16, SEQ ID NOs: 19 and 20, SEQ ID NOs: 21 and 22, SEQ ID NOs: 1 and 2; SEQ ID NOs: 3 and 4; SEQ ID NOs: 5 and 6; SEQ ID NOs: 9 and 10; SEQ ID NOs: 11 and 12; SEQ ID NOs: 13 and 14, and a primer set listed in Tables 2, 3, 4 or 5.

11. A method of isolating a fetal nucleic acid sample comprising:
isolating, by size fractionation, a nucleic acid sample consisting essentially of polynucleotides of about 50 base pairs to about 300 base pairs in length from a cervical mucus sample obtained from a female subject containing the fetus.

## Patentansprüche

1. Verfahren zur Durchführung eines genetischen Tests an einem Fötus, das Folgendes umfasst:
das Isolieren einer Nucleinsäureprobe aus einer Zervikalschleimprobe von einem weiblichen Individuum, das den Fötus enthält, durch Größenfraktionierung, wobei die Nucleinsäureprobe im Wesentlichen aus Polynucleotiden in einem Größenbereich von etwa 50 Basenpaaren bis etwa 300 Basenpaaren besteht, und
wobei das Ergebnis eines genetischen Tests an der Nucleinsäureprobe die genetische Zusammensetzung des Fötus angibt.

2. Verfahren nach Anspruch 1, wobei die Zervikalschleimprobe durch transzervikale Abstriche, endozervikale Spülung, Cytobrush, Aspiration, intrauterine Spülung oder eine Kombination daraus erhalten wird.

3. Verfahren nach Anspruch 1, wobei die Nucleinsäureprobe eine DNA-Probe oder RNA-Probe ist.

4. Verfahren nach Anspruch 1, das weiters die Verwendung der isolierten Nucleinsäureprobe zum Testen auf eine genetische Zusammensetzung umfasst, die nicht ausschließlich mit einem Y-Chromosom assoziiert ist, wobei die genetische Zusammensetzung der isolierten Nucleinsäureprobe die genetische Zusammensetzung des Fötus angibt.

5. Verfahren nach Anspruch 4, wobei die genetische Zusammensetzung aus der aus Trisomie, partieller Trisomie, chromosomaler Verdoppelung, Monosomie, partieller Monosomie, chromosomaler Deletion, chromosomaler Translokation und chromosomaler Inversion bestehenden Gruppe ausgewählt ist.

6. Verfahren nach Anspruch 4, wobei die genetische Zusammensetzung einen Erkrankungszustand anzeigt, der aus der aus Down-Syndrom, Edwards-Syndrom, Pätau-Syndrom, Fragiles-X-Syndrom, Turner-Syndrom, Klinefelter-Syndrom, Tripel-X-Syndrom, XYY-Syndrom, Trisomie 8, Trisomie 16, Wolf-Hirschhorn-Syndrom und RhD-Syndrom bestehenden Gruppe ausgewählt ist.

7. Verfahren nach Anspruch 1, das weiters die Verwendung der isolierten Nucleinsäureprobe als Matrize für einen genetischen Test unter Verwendung einer Testtechnologie umfasst, die aus der aus PCR, Echtzeit-PCR, LCR, Q-B-Replicase, SDA, RCA, TMA, LADA, MDA und Invader bestehenden Gruppe ausgewählt sind.

8. Verfahren nach Anspruch 1, das weiters die Verwendung der isolierten Nucleinsäureprobe zum Testen auf die Gegenwart eines genetischen Markers oder eines Allels im Fötus umfasst, wobei die Gegenwart des genetischen Markers oder Allels auf der Amplifikation eines Nucleotidfragments unter Verwendung eines Primerpaars basiert, das für den genetischen Marker oder das Allel spezifisch ist.

9. Verfahren nach Anspruch 8, wobei das Allel einem genetischen Zustand entspricht, der aus der aus Sichelzellenanämie, Phenylketonurie, Tay-Sachs-Krankheit, zystischer Fibrose, β-Thalassämie, adrenaler Hyperplasie, Fanconi-Anämie, spinaler Muskelatrophie, Duchenne-Muskeldystrophie, Huntington-Chorea, Dystrophia myotonica, Robertson-Translokation, Angelman-Syndrom, DiGeorge-Syndrom, tuberöser Sklerose, Ataxia telangiectasia und Prader-Willi-Syndrom bestehenden Gruppe ausgewählt ist.

10. Verfahren nach Anspruch 8, wobei das Primerpaar aus der aus Primer der Seq.-ID Nr. 17 und 18, Seq.-ID Nr. 15 und 16, Seq.-ID Nr. 19 und 20, Seq.-ID Nr. 21 und 22, Seq.-ID Nr. 1 und 2, Seq.-ID Nr. 3 und 4, Seq.-ID Nr. 5 und 6, Seq.-ID Nr. 9 und 10, Seq.-ID Nr. 11 und 12, Seq.-ID Nr. 13 und 14 und einem in Tabelle 2, 3, 4 oder 5 angeführten Primerset bestehenden Gruppe ausgewählt ist.

11. Verfahren zur Isolation einer fötalen Nucleinsäureprobe, das Folgendes umfasst:
das Isolieren einer Nucleinsäureprobe, die im Wesentlichen aus Polynucleotiden mit einer Länge von etwa 50 Basenpaaren bis etwa 300 Basenpaaren besteht, aus einer Zervikalschleimprobe von einem weiblichen Individuum, das den Fötus enthält, durch Größenfraktionierung.

## Revendications

1. Procédé pour réaliser un test génétique d'un foetus, qui consiste à
isoler, par fractionnement de taille, un échantillon d'acide nucléique à partir d'un échantillon de glaire cervicale obtenu chez un sujet féminin contenant le foetus, l'échantillon d'acide nucléique consistant essentiellement en des polynucléotides ayant une taille comprise entre environ 50 paires de bases et environ 300 paires de bases, et
dans lequel le résultat d'un test génétique sur l'échantillon d'acide nucléique indique une composition génétique du foetus.

2. Procédé selon la revendication 1, dans lequel l'échantillon de glaire cervicale est obtenu par un prélèvement transcervical, un lavage endocervical, une cytobrosse, une aspiration, un lavage intra-utérin, ou une combinaison de ceux-ci.

3. Procédé selon la revendication 1, dans lequel l'échantillon d'acide nucléique est un échantillon d'ADN ou un échantillon d'ARN.

4. Procédé selon la revendication 1, qui consiste en outre à utiliser l'échantillon d'acide nucléique isolé afin de tester une composition génétique qui n'est pas uniquement associée au chromosome Y, où la composition génétique de l'échantillon d'acide nucléique isolé indique la composition génétique du foetus.

5. Procédé selon la revendication 4, dans lequel la composition génétique est sélectionnée dans le groupe consistant en une trisomie, une trisomie partielle, une duplication chromosomique, une monosomie, une monosomie partielle, une délétion chromosomique, une translocation chromosomique, et une inversion chromosomique.

6. Procédé selon la revendication 4, dans lequel la composition génétique indique un état pathologique sélectionné dans le groupe consistant en le syndrome de Down, le syndrome d'Edwards, le syndrome de Patau, le syndrome de l'X fragile, le syndrome de Turner, le syndrome de Klinefelter, le syndrome triple X, le syndrome XYY, la trisomie 8, la trisomie 16, le syndrome de Wolf-Hirschhorn, et le syndrome RhD.

7. Procédé selon la revendication 1, qui consiste en outre à utiliser l'échantillon d'acide nucléique isolé en tant que matrice pour un test génétique en utilisant une technologie d'analyse sélectionnée dans le groupe consistant en une PCR, une PCR en temps réel, une LCR, une amplification par Q-bêta réplicase, une SDA, une RCA, une TMA, une LADA, une MDA, et la technologie Invader.

8. Procédé selon la revendication 1, qui consiste en outre à utiliser l'échantillon d'acide nucléique isolé afin de tester la présence d'un marqueur génétique ou d'un allèle chez le foetus, où la présence du marqueur génétique ou de l'allèle est basée sur l'amplification d'un fragment nucléotidique en utilisant une paire d'amorces spécifique pour le marqueur génétique ou l'allèle.

9. Procédé selon la revendication 8, dans lequel l'allèle correspond à une maladie génétique sélectionnée dans le groupe consistant en la drépanocytose, la phénylcétonurie, la maladie de Tay-Sachs, la fibrose kystique, la bêta-thalassémie, l'hyperplasie surrénalienne, l'anémie de Fanconi, l'amyotrophie spinale, la dystrophie musculaire de Duchenne, la maladie de Huntington, la dystrophie myotonique, une translocation robertsonienne, le syndrome d'Angelman, le syndrome de DiGeorge, la sclérose tubéreuse, l'ataxie-télangiectasie, et le syndrome de Prader-Willi.

10. Procédé selon la revendication 8, dans lequel la paire d'amorces est sélectionnée dans le groupe consistant en les amorces de SEQ ID NO: 17 et 18, SEQ ID NO: 15 et 16, SEQ ID NO: 19 et 20, SEQ ID NO: 21 et 22, SEQ ID NO: 1 et 2; SEQ ID NO: 3 et 4; SEQ ID NO: 5 et 6; SEQ ID NO: 9 et 10; SEQ ID NO: 11 et 12; SEQ ID NO: 13 et 14, et un jeu d'amorces listé dans les Tableaux 2, 3, 4 ou 5.

11. Procédé pour isoler un échantillon d'acide nucléique foetal, qui consiste à:
isoler, par fractionnement de taille, un échantillon d'acide nucléique consistant essentiellement en des polynucléotides d'une longueur d'environ 50 paires de bases à environ 300 paires de bases à partir d'un échantillon de glaire cervicale obtenu chez un sujet féminin contenant le foetus.
